# EUROPEAN PATENT APPLICATION

(11) **EP 2 287 940 A1**
(43) Date of publication of application: **23.02.2011**
(21) Application number: 09735980.6
(22) Date of filing: 24.04.2009
(51) Int. Cl.: H01L 51/50, C07D 213/06, C07D 251/24, C08K 5/3432, C08K 5/3492, C08L 65/00, C09K 11/06

(54) **COMPOSITION CONTAINING NITROGENOUS HETEROCYCLIC COMPOUNDS**

(30) Priority: 25.04.2008 JP 2008115199; 30.10.2008 JP 2008279739
(71) Applicant: Sumitomo Chemical Company, Limited, Tokyo 104-8260 (JP); Sumation Co., Ltd., Chuo-ku Tokyo 104-8260 (JP)
(72) Inventor: ANRYU, Makoto, Tsukuba-shi Ibaraki 305-0821 (JP); SOGA, Masayuki, Tsukuba-shi Ibaraki 305-0821 (JP)
(74) Representative: Duckworth, Timothy John
(86) International application number: PCT/JP2009/058601
(87) International publication number: WO 2009/131254

(57) **Abstract**

A composition comprising a compound represented by the following formula (1): 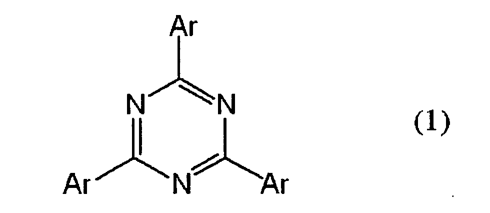 wherein Ar represents an aryl group or a mono-valent heterocyclic group.)
or a compound having a residue of a compound represented by the above-described formula (1), and
a compound represented by the following formula (2): 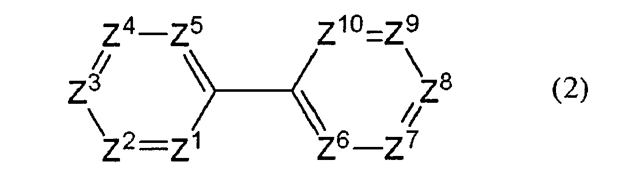 wherein one member of Z¹ to Z³ represents -N= and two remainders thereof each represent -C(R')=; Z⁴ and Z⁵ each represent -C(R')=; one member of Z⁶ to Z⁸ represents -N= and two remainders thereof each represent -C(R')=; Z⁹ and Z¹⁰ each represent -C(R')=; R' represents a substituent such as a hydrogen atom, an alkyl group, an alkoxy group,
an aryl group, an aryloxy group and the like.
or a compound having a residue of a compound represented by the above-described formula (2).

## Description

### Technical Field

The present invention relates to a composition comprising a nitrogen-containing heterocyclic compound and an organic electroluminescent device using the same.

### Background Art

Recently, an organicelectroluminescent display using an organic electroluminescent device is attracting attention, as the next generation display. This organic electroluminescent device has organic layers such as a light emitting layer, a charge transporting layer and the like. For the above-described organic layers, organic materials excellent in electron injectability are required, and a triazine derivative is suggested as an example thereof (JP-A No. 8-119163, JP-A No. 2007-137829).

### Disclosure of the Invention

However, when this triazine derivative is solely used in the above-described organic layer, the luminance half life of the resultant organic electroluminescent device is not necessarily long.

An object of the present invention is to provide an organic material which is capable of giving an organic electroluminescent device having a long luminance half life when used for production of an organic electroluminescent device.

The present invention provides, in a first aspect, a composition comprising:
a compound represented by the following formula (1): 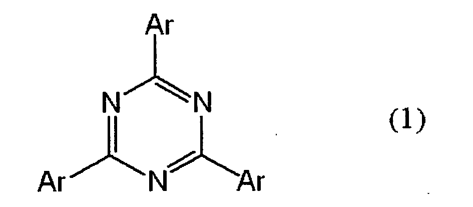 wherein each Ar represents an aryl group optionally having a substituent, or a mono-valent heterocyclic group optionally having a substituent;, where three Ars may be the same or different,
   or a compound having a residue of a compound represented by the above-described formula (1), and
a compound represented by the following formula (2): 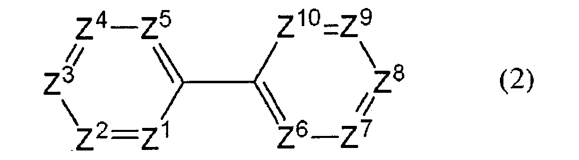 wherein one member of Z¹, Z² and Z³ represents -N= and two remainders thereof each represent -C(R')=; Z⁴ and Z⁵ each represent -C(R')=; one member of Z⁶, Z⁷ and Z⁸ represents -N= and two remainders thereof each represent -C(R')=; Z⁹ and Z¹⁰ each represent -C(R')=; R' represents a hydrogen atom, an alkyl group optionally having a substituent, an alkoxy group optionally having a substituent, an alkylthio group optionally having a substituent, an aryl group optionally having a substituent, an aryloxy group optionally having a substituent, an arylthio group optionally having a substituent, an alkenyl group optionally having a substituent, an alkynyl group optionally having a substituent, an amino group optionally having a substituent, a silyl group optionally having a substituent, a halogen atom, an acyl group optionally having a substituent, an acyloxy group optionally having a substituent, a mono-valent heterocyclic group optionally having a substituent, a heterocyclic thio group optionally having a substituent, an imine residue, an amide group optionally having a substituent, an acid imide group, carboxyl group, a nitro group, or a cyano group; eight -C(R')= groups may be the same or different; when Z² and Z³ each represent -C(R')=, two R' s contained in Z² and Z³ may be combined together to form a benzene ring, and when Z³ represents -C(R')=, two R' s contained in Z³ and Z⁴ may be combined together to form a benzene ring and two R's contained in Z⁴ and Z⁵ may be combined together to form a benzene ring, providing that two or more combinations of Z² and Z³, Z³ and Z⁴, and Z⁴ and Z⁵ do not simultaneously form a benzene ring; when Z⁷ and Z⁸ each represent -C(R')=, two R's contained in Z⁷ and Z⁸ may be combined together to form a benzene ring, and when Z⁸ represents -C(R')=, two R's contained in Z⁸ and Z⁹ may be combined together to form a benzene ring and two R's contained in Z⁹ and Z¹⁰ may be combined together to form a benzene ring, providing that two or more combinations of Z⁷ and Z⁸, Z⁸ and Z⁹, and Z⁹ and Z¹⁰ do not simultaneously form a benzene ring; the benzene ring formed by mutual combination of two R's optionally has a substituent. or a compound having a residue of a compound represented by the above-described formula (2).

The present invention provides, in a second aspect, an organic electroluminescent device obtained by using the above-described composition.

The present invention provides, in a third aspect, a planar light source and a display comprising the above-described organic electroluminescent device.

The present invention provides, in a fourth aspect, a film obtained by using the above-described composition.

The present invention provides, in a fifth aspect, a method of producing an organic electroluminescent device comprising heating the above-described film at 50 to 200°C.

### Modes for Carrying out the Invention

### <Explanation of terms>

The terms commonly used in the present specification will be explained below. In the present specification, Me means a methyl group, t-Bu means a tert-butyl group, and Ph manes a phenyl group.

The halogen atom includes a fluorine atom, chlorine atom, bromine atom and iodine atom.

The term "Cₓ to C_{y}" (x and y are positive integers satisfying x<y) represents that the carbon atom number of an organic group described together with this term is x to y.

The alkyl group optionally has a substituent such as an alkyl group, alkoxy group, alkylthio group, aryl group, aryloxy group, arylthio group, alkenyl group, alkynyl group, amino group, silyl group, halogen atom, acyl group, acyloxy group, mono-valent heterocyclic group, heterocyclic thio group, imine residue, amide group, acid imide group, carboxyl group, nitro group, cyano group and the like (hereinafter, when referred to "substituent", the same meaning is indicated unless otherwise stated), means usually an unsubstituted alkyl group and an alkyl group substituted by a halogen atom and the like, and includes both linear alkyl groups and cyclic alkyl groups (cycloalkyl groups). The alkyl group may be branched. The alkyl group has a carbon atom number of usually 1 to 20, preferably 1 to 15, more preferably about 1 to 10. Examples of the alkyl group include a methyl group, ethyl group, propyl group, isopropyl group, butyl group, isobutyl group, s-butyl group, t-butyl group, pentyl group, isoamyl group, hexyl group, cyclohexyl group, heptyl group, octyl group, 2-ethylhexyl group, nonyl group, decyl group, 3,7-dimethyloctyl group, dodecyl group, trifluoromethyl group, pentafluoroethyl group, perfluorobutyl group, perfluorohexyl group, perfluorooctyl group, and the like. Examples of the C₁ to C₁₂ alkyl group include a methyl group, ethyl group, propyl group, isopropyl group, butyl group, isobutyl group, s-butyl group, t-butyl group, pentyl group, isoamyl group, hexyl group, cyclohexyl group, heptyl group, octyl group, nonyl group, decyl group and dodecyl group.

The alkoxy group optionally has a substituent, means usually an unsubstituted alkoxy group and an alkoxy group substituted with a halogen atom, alkoxy group or the like, and includes both linear alkoxy groups and cyclic alkoxy groups (cycloalkoxy groups). The alkoxy group may be branched. The alkoxy group has a carbon atom number of usually 1 to 20, preferably 1 to 15, more preferably about 1 to 10. Examples of the alkoxy group include a methoxy group, ethoxy group, propyloxy group, isopropyloxy group, butoxy group, isobutoxy group, s-butoxy group, t-butoxy group, pentyloxy group, hexyloxy group, cyclohexyloxy group, heptyloxy group, octyloxy group, 2-ethylhexyloxy group, nonyloxy group, decyloxy group, 3,7-dimethyloctyloxy group, dodecyloxy group, trifluoromethoxy group, pentafluoroethoxy group, perfluorobutoxy group, perfluorohexyl group, perfluorooctyl group, methoxymethyloxy group, and 2-methoxyethyloxy group.

Examples of the C₁ to C₁₂ alkoxy group include a methoxy group, ethoxy group, propyloxy group, isopropyloxy group, butoxy group, isobutoxy group, s-butoxy group, t-butoxy group, pentyloxy group, hexyloxy group, cyclohexyloxy group, heptyloxy group, octyloxy group, 2-ethylhexyloxy group, nonyloxy group, decyloxy group, 3,7-dimethyloctyloxy group, and dodecyloxy group.

The alkylthio group optionally has a substituent, means usually an unsubstituted alkylthio group and an alkylthio group substituted with a halogen atom or the like, and includes both linear alkylthio groups and cyclic alkylthio groups (cycloalkylthio groups). The alkylthio group may be branched. The alkylthio group has a carbon atom number of usually 1 to 20, preferably 1 to 15, more preferably about 1 to 10. Examples of the alkylthio group include a methylthio group, ethylthio group, propylthio group, isopropylthio group, butylthio group, isobutylthio group, s-butylthio group, t-butylthio group, pentylthio group, hexylthio group, cyclohexylthio group, heptylthio group, octylthio group, 2-ethylhexylthio group, nonylthio group, decylthio group, 3,7-dimethyloctylthio group, dodecylthio group, and trifluoromethylthio group. Examples of the C₁ to C₁₂ alkylthio group include a methylthio group, ethylthio group, propylthio group, isopropylthio group, butylthio group, isobutylthio group, s-butylthio group, t-butylthio group, pentylthio group, hexylthio group, cyclohexylthio group, heptylthio group, octylthio group, 2-ethylhexylthio group, nonylthio group, decylthio group, 3,7-dimethyloctylthio group, and dodecylthio group.

The aryl group is an atomic group remaining after removing, from an aromatic hydrocarbon, one hydrogen atom connected to a carbon atom constituting the aromatic ring, optionally has a substituent, and means usually an unsubstituted aryl group and an aryl group substituted with a halogen atom, alkoxy group, alkyl group or the like. The aryl group also includes those having a condensed ring, and those having two or more independent benzene rings or condensed rings connected via a single bond or a di-valent organic group, for example, an alkenylene group such as a vinylene group and the like. The aryl group has a carbon atom number of usually 6 to 60, preferably 6 to 48, more preferably about 6 to 30. Examples of the aryl group include a phenyl group, C₁ to C₁₂ alkoxyphenyl groups, C₁ to C₁₂ alkylphenyl groups, 1-naphthyl group, 2-naphthyl group, 1-anthracenyl group, 2-anthracenyl group, 9-anthracenyl group, pentafluorophenyl group, biphenyl group, C₁ to C₁₂ alkoxybiphenyl groups, and C₁ to C₁₂ alkylbiphenyl groups, and of them, preferable are a phenyl group, C₁ to C₁₂ alkoxyphenyl groups, C₁ to C₁₂ alkylphenyl groups, biphenyl group, C₁ to C₁₂ alkoxybiphenyl groups, and C₁ to C₁₂ alkylbiphenyl groups.

Examples of the C₁ to C₁₂ alkoxyphenyl group include a methoxyphenyl group, ethoxyphenyl group, propyloxyphenyl group, isopropyloxyphenyl group, butyloxyphenyl group, isobutyloxyphenyl group, t-butyloxyphenyl group, pentyloxyphenyl group, hexyloxyphenyl group, and octyloxyphenyl group.

Examples of the C₁ to C₁₂ alkylphenyl group include a methylphenyl group, ethylphenyl group, dimethylphenyl group, propylphenyl group, mesityl group, methylethylphenyl group, isopropylphenyl group, butylphenyl group, isobutylphenyl group, t-butylphenyl group, pentylphenyl group, isoamylphenyl group, hexylphenyl group, heptylphenyl group, octylphenyl group, nonylphenyl group, decylphenyl group, and dodecylphenyl group.

The aryloxy group optionally has a substituent, and means usually an unsubstituted aryloxy group and an aryloxy group substituted with a halogen atom, alkoxy group, alkyl group or the like. The aryloxy group has a carbon atom number of usually 6 to 60, preferably 6 to 48, more preferably 6 to 30. Examples of the aryloxy group include a phenoxy group, C₁ to C₁₂ alkoxyphenoxy groups, C₁ to C₁₂ alkylphenoxy groups, 1-naphthyloxy group, 2-naphthyloxy group, and pentafluorophenyloxy group, and of them, preferable are C₁ to C₁₂ alkoxyphenoxy groups and C₁ to C₁₂ alkylphenoxy groups.

Examples of the C₁ to C₁₂ alkoxyphenoxy group include a methoxyphenoxy group, ethoxyphenoxy group, propyloxyphenoxy group, isopropyloxyphenoxy group, butyloxyphenoxy group, isobutyloxyphenoxy group, t-butyloxyphenoxy group, pentyloxyphenoxy group, hexyloxyphenoxy group, and octyloxyphenoxy group.

Examples of the C₁ to C₁₂ alkylphenoxy group include a methylphenoxy group, ethylphenoxy group, dimethylphenoxy group, propylphenoxy group, 1,3,5-trimethylphenoxy group, methylethylphenoxy group, isopropylphenoxy group, butylphenoxy group, isobutylphenoxy group, s-butylphenoxy group, t-butylphenoxy group, pentylphenoxy group, isoamylphenoxy group, hexylphenoxy group, heptylphenoxy group, octylphenoxy group, nonylphenoxy group, decylphenoxy group, and dodecylphenoxy group.

The arylthio group optionally has a substituent, and means usually an unsubstituted arylthio group and an arylthio group substituted with a halogen atom, alkoxy group, alkyl group or the like. The arylthio group has a carbon atom number of usually 6 to 60, preferably 6 to 48, more preferably 6 to 30. Examples of the arylthio group include a phenylthio group, C₁ to C₁₂ alkoxyphenylthio groups, C₁ to C₁₂ alkylphenylthio groups, 1-naphthylthio group, 2-naphthylthio group, and pentafluorophenylthio group.

The arylalkyl group optionally has a substituent, and means usually an unsubstituted arylalkyl group and an arylalkyl group substituted with a halogen atom, alkoxy group, alkyl group or the like. The arylalkyl group has a carbon atom number of usually 7 to 60, preferably 7 to 48, more preferably 7 to 30. Examples of the arylalkyl group include phenyl-C₁ to C₁₂ alkyl groups, C₁ to C₁₂ alkoxyphenyl-C₁ to C₁₂ alkyl groups, C₁ to C₁₂ alkylphenyl-C₁ to C₁₂ alkyl groups, 1-naphthyl-C₁ to C₁₂ alkyl groups, and 2-naphthyl-C₁ to C₁₂ alkyl groups.

The arylalkoxy group optionally has a substituent, and means usually an unsubstituted arylalkoxy group and an arylalkoxy group substituted with a halogen atom, alkoxy group, alkyl group or the like. The arylalkoxy group has a carbon atom number of usually 7 to 60, preferably 7 to 48, more preferably 7 to 30. Examples of the arylakoxy group include phenyl-C₁ to C₁₂ alkoxy groups, C₁ to C₁₂ alkoxyphenyl-C₁ to C₁₂ alkoxy groups, C₁ to C₁₂ alkylphenyl-C₁ to C₁₂ alkoxy groups, 1-naphthyl-C₁ to C₁₂ alkoxy groups, and 2-naphthyl-C₁ to C₁₂ alkoxy groups.

The arylalkylthio group optionally has a substituent, and means usually an unsubstituted arylalkylthio group and an arylalkylthio group substituted with a halogen atom, alkoxy group, alkyl group or the like. The arylalkylthio group has a carbon atom number of usually 7 to 60, preferably 7 to 48, more preferably 7 to 30. Examples of the arylalkylthio group include phenyl-C₁ to C₁₂ alkylthio groups, C₁ to C₁₂ alkoxyphenyl-C₁ to C₁₂ alkylthio groups, C₁ to C₁₂ alkylphenyl-C₁ to C₁₂ alkylthio groups, 1-naphthyl-C₁ to C₁₂ alkylthio groups, and 2-naphthyl-C₁ to C₁₂ alkylthio groups.

The alkenyl group optionally has a substituent, and includes linear alkenyl groups, branched alkenyl groups and cyclic alkenyl groups. The alkenyl group has a carbon atom number of usually 2 to 20, preferably 2 to 15, more preferably 2 to 10. Examples of the alkenyl group include a vinyl group, 1-propenyl group, 2-propenyl group, 1-butenyl group, 2-butenyl group, 1-pentenyl group, 2-pentenyl group, 1-hexenyl group, 2-hexenyl group, and 1-octenyl group.

The arylalkenyl group optionally has a substituent, and means usually an unsubstituted arylalkenyl group and an arylalkenyl group substituted with a halogen atom, alkoxy group, alkyl group or the like. The arylalkenyl group has a carbon atom number of usually 8 to 60, preferably 8 to 48, more preferably 8 to 30. Examples of the arylalkenyl group include phenyl-C₂ to C₁₂ alkenyl groups, C₁ to C₁₂ alkoxyphenyl-C₂ to C₁₂ alkenyl groups, C₁ to C₁₂ alkylphenyl-C₂ to C₁₂ alkenyl groups, 1-naphthyl-C₂ to C₁₂ alkenyl groups, and 2-naphthyl-C₂ to C₁₂ alkenyl groups, and of them, preferable are C₁ to C₁₂ alkoxyphenyl-C₂ to C₁₂ alkenyl groups and C₂ to C₁₂ alkylphenyl-C₂ to C₁₂ alkenyl groups.

Examples of the C₂ to C₁₂ alkenyl group include a vinyl group, 1-propenyl group, 2-propenyl group, 1-butenyl group, 2-butenyl group, 1-pentenyl group, 2-pentenyl group, 1-hexenyl group, 2-hexenyl group and 1-octenyl group.

The alkynyl group optionally has a substituent, and includes linear alkynyl groups and branched alkynyl groups. The alkynyl group has a carbon atom number of usually 2 to 20, preferably 2 to 15, more preferably 2 to 10. Examples of the alkynyl group include an ethynyl group, 1-propynyl group, 2-propynyl group, 1-butynyl group, 2-butynyl group, 1-pentynyl group, 2-pentynyl group, 1-hexynyl group, 2-hexynyl group and 1-octynyl group.

The arylalkynyl group optionally has a substituent, and means usually an unsubstituted arylalkynyl group and an arylalkynyl group substituted with a halogen atom, alkoxy group, alkyl group or the like. The arylalkynyl group has a carbon atom number of usually 8 to 60, preferably 8 to 48, more preferably 8 to 30. Examples of the arylalkynyl group include phenyl-C₂ to C₁₂ alkynyl groups, C₁ to C₁₂ alkoxyphenyl-C₂ to C₁₂ alkynyl groups, C₁ to C₁₂ alkylphenyl-C₂ to C₁₂ alkynyl groups, 1-naphthyl-C₂ to C₁₂ alkynyl groups, and 2-naphthyl-C₂ to C₁₂ alkynyl groups, and of them, preferable are C₁ to C₁₂ alkoxyphenyl-C₂ to C₁₂ alkynyl groups and C₁ to C₁₂ alkylphenyl-C₂ to C₁₂ alkynyl groups.

Examples of the C₂ to C₁₂ alkynyl group include an ethynyl group, 1-propynyl group, 2-propynyl group, 1-butynyl group, 2-butynyl group, 1-pentynyl group, 2-pentynyl group, 1-hexynyl group, 2-hexynyl group and 1-octynyl group.

The mono-valent heterocyclic group indicates an atomic group remaining after removal of one hydrogen atom from a heterocyclic compound (particularly, aromatic heterocyclic compound), optionally has a substituent, and means usually an unsubstituted mono-valent heterocyclic group and a mono-valent heterocyclic group substituted with a substituent such as an alkyl group or the like. The mono-valent heterocyclic group has a carbon atom number of usually 4 to 60, preferably 4 to 30, more preferably 4 to 20, not including the carbon atom number of the substituent. The heterocyclic compound includes organic compounds having a cyclic structure in which elements constituting the ring include not only a carbon atom but also a hetero atom such as an oxygen atom, sulfur atom, nitrogen atom, phosphorus atom, boron atom, silicon atom, selenium atom, tellurium atom, arsenic atom and the like. Examples of the mono-valent heterocyclic group include a thienyl group, C₁ to C₁₂ alkylthienyl groups, pyrrolyl group, furyl group, pyridyl group, C₁ to C₁₂ alkylpyridyl groups, pyridazinyl group, pyrimidyl group, pyrazinyl group, triazinyl group, pyrrolidyl group, piperidyl group, quinolyl group, and isoquinolyl group, and of them, preferable are a thienyl group, C₁ to C₁₂ alkylthienyl groups, pyridyl group and C₁ to C₁₂ alkylpyridyl groups.

The heterocyclicthio group means a group obtained by substituting a hydrogen atom of a mercapto group with a mono-valent heterocyclic group, and optionally has a substituent. Examples of the heterocyclicthio group include heteroarylthio groups such as a pyridylthio group, pyridazinylthio group, pyrimidylthio group, pyrazinylthio group, triazinylthio group and the like.

The amino group optionally has a substituent, and means usually an unsubstituted amino group and an amino group substituted with one or two substituents selected from the group consisting of an alkyl group, aryl group, arylalkyl group and mono-valent heterocyclic group (hereinafter, referred to as "substituted amino group"). The substituent optionally further has a substituent (hereinafter, referred to as "secondary substituent", in some cases). The substituted amino group has a carbon atom number of usually 1 to 60, preferably 2 to 48, more preferably 2 to 40, not including the carbon atom number of the secondary substituent. Examples of the substituted amino group include a methylamino group, dimethylamino group, ethylamino group, diethylamino group, propylamino group, dipropylamino group, isopropylamino group, diisopropylamino group, butylamino group, isobutylamino group, s-butylamino group, t-butylamino group, pentylamino group, hexylamino group, heptylamino group, octylamino group, 2-ethylhexylamino group, nonylamino group, decylamino group, 3,7-dimethyloctylamino group, dodecylamino group, cyclopentylamino group, dicyclopentylamino group, cyclohexylamino group, dicyclohexylamino group, ditrifluoromethylamino group, phenylamino group, diphenylamino group, C₁ to C₁₂ alkoxyphenylamino groups, di(C₁ to C₁₂ alkoxyphenyl)amino groups, C₁ to C₁₂ alkylphenylamino groups, di(C₁ to C₁₂ alkylphenyl)amino groups, 1-naphthylamino group, 2-naphthylamino group, pentafluorophenylamino group, pyridylamino group, pyridazinylamino group, pyrimidylamino group, pyrazinylamino group, triazinylamino group, phenyl-C₁ to C₁₂ alkylamino groups, C₁ to C₁₂ alkoxyphenyl-C₁ to C₁₂ alkylamino groups, di (C₁ to C₁₂ alkoxyphenyl-C₁ to C₁₂ alkyl)amino groups, C₁ to C₁₂ alkylphenyl-C₁ to C₁₂ alkylamino groups, di(C₁ to C₁₂ alkylphenyl-C₁ to C₁₂ alkyl)amino groups, 1-naphthyl-C₁ to C₁₂ alkylamino groups and 2-naphthyl-C₁ to C₁₂ alkylamino groups.

The silyl group optionally has a substituent, and means usually an unsubstituted silyl group and a silyl group substituted with one, two or three substituents selected from the group consisting of an alkyl group, aryl group, arylalkyl group and mono-valent heterocyclic group (hereinafter, referred to as "substituted silyl group"). The substituent optionally has a secondary substituent. The substituted silyl group has a carbon atom number of usually 1 to 60, preferably 3 to 48, more preferably 3 to 40, not including the carbon atom number of the secondary substituent.

Examples of the substituted silyl group include a trimethylsilyl group, triethylsilyl group, tripropylsilyl group, tri-isopropylsilyl group, dimethyl-isopropylsilyl group, diethyl-isopropylsilyl group, t-butyldimethylsilyl group, pentyldimethylsilyl group, hexyldimethylsilyl group, heptyldimethylsilyl group, octyldimethylsilyl group, 2-ethylhexyl-dimethylsilyl group, nonyldimethylsilyl group, decyldimethylsilyl group, 3,7-dimethyloctyl-dimethylsilyl group, dodecyldimethylsilyl group, phenyl-C₁ to C₁₂ alkylsilyl groups, C₁ to C₁₂ alkoxyphenyl-C₁ to C₁₂ alkylsilyl groups, C₁ to C₁₂ alkylphenyl-C₁ to C₁₂ alkylsilyl groups, 1-naphthyl-C₁ to C₁₂ alkylsilyl groups, 2-naphthyl-C₁ to C₁₂ alkylsilyl groups, phenyl-C₁ to C₁₂ alkyldimethylsilyl groups, triphenylsilyl group, tri-p-xylylsilyl group, tribenzylsilyl group, diphenylmethylsilyl group, t-butyldiphenylsilyl group and dimethylphenylsilyl group.

The acyl group optionally has a substituent, and means usually an unsubstituted acyl group and an acyl group substituted with a halogen atom or the like. The acyl group has a carbon atom number of usually 2 to 20, preferably 2 to 18, more preferably 2 to 16. Examples of the acyl group include an acetyl group, propionyl group, butylyl group, isobutylyl group, pivaloyl group, benzoyl group, trifluoroacetyl group and pentafluorobenzoyl group.

The acyloxy group optionally has a substituent, and means usually an unsubstituted acyloxy group and an acyloxy group substituted with a halogen atom or the like. The acyloxy group has carbon atom number of usually 2 to 20, preferably 2 to 18, more preferably 2 to 16. Examples of the acyloxy group include an acetoxy group, propionyloxy group, butylyloxy group, isobutylyloxy group, pivaloyloxy group, benzoyloxy group, trifluoroacetyloxy group, pentafluorobenzoyloxy group and the like.

The imine residue means a residue obtained by removing, from an imine compound having a structure represented by at least one of the formula: H-N=C< and the formula: -N=CH-, one hydrogen atom in this structure. Examples of such an imine compound include aldimines and ketimines, and compounds obtained by substitution of a hydrogen atom connected to a nitrogen atom in aldimines with an alkyl group, aryl group, arylalkyl group, arylalkenyl group, arylalkynyl group or the like. The imine residue has carbon atom number of usually 2 to 20, preferably 2 to 18, more preferably 2 to 16. Examples of the imine residue include groups represented by the general formula: -CR^{X}=N-R^{Y} or the general formula: -N=C(R^{Y})₂ wherein R^{X} represents a hydrogen atom, alkyl group, aryl group, arylalkyl group, arylalkenyl group or arylalkynyl group, R^{Y} represents an alkyl group, aryl group, arylalkyl group, arylalkenyl group or arylalkynyl group. When there exist two R^{Y}s, they may be the same or different, and two R^{Y}s may be mutually connected and integrated to form a ring as a di-valent group, for example, an alkylene group having 2 to 18 carbon atoms such as an ethylene group, trimethylene group, tetramethylene group, pentamethylene group, hexamethylene group and the like.). Examples of the imine residue include groups of the following structural formulae, and the like.

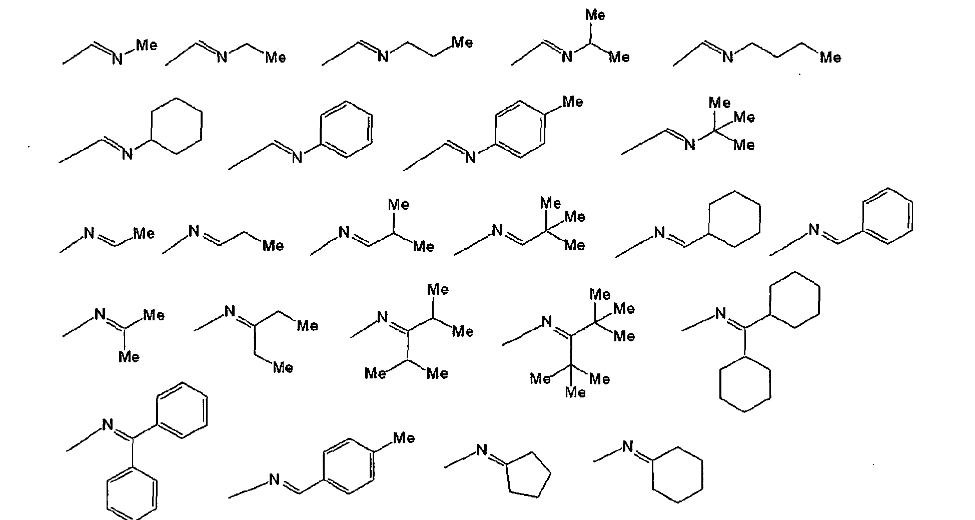

The amide group optionally has a substituent, and means usually an unsubstituted amide group and an amide group substituted with a halogen atom or the like. The amide group has a carbon atom number of usually 2 to 20, preferably 2 to 18, more preferably 2 to 16. Examples of the amide group include a formamide group, acetamide group, propioamide group, butyroamide group, benzamide group, trifluoroacetamide group, pentafluorobenzamide group, diformamide group, diacetamide group, dipropioamide group, dibutyroamide group, dibenzamide group, ditrifluoroacetamide group, dipentafluorobenzamide group and the like.

The acid imide group means a residue obtained by removing from an acid imide one hydrogen atom connected to its nitrogen atom. The acid imide group has a carbon atom number of usually 4 to 20, preferably 4 to 18, more preferably 4 to 16. Examples of the acid imide group include groups shown below.

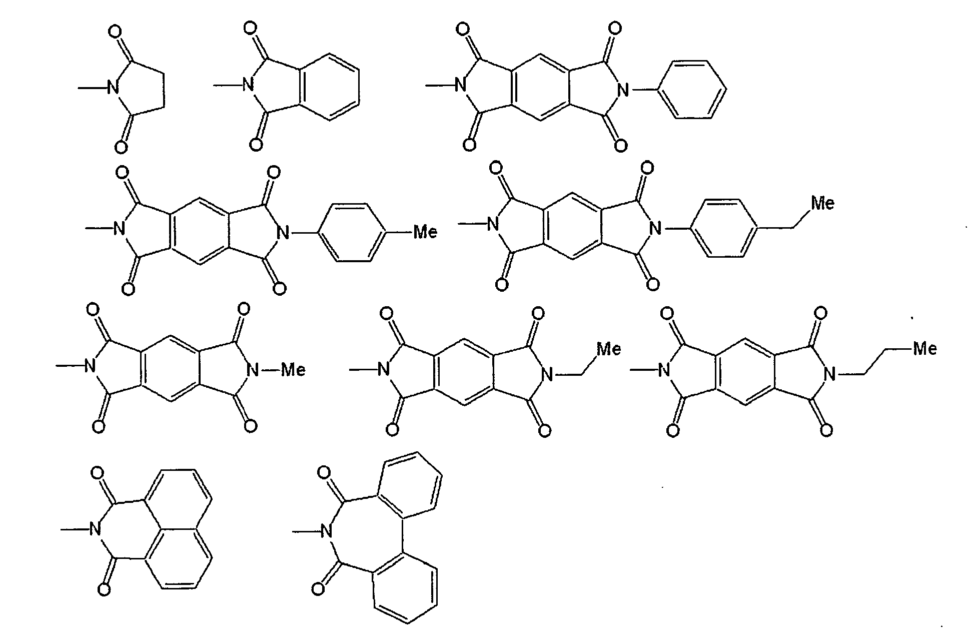

The arylene group means an atomic group obtained by removing two hydrogen atoms from an aromatic hydrocarbon, and includes those having an independent benzene ring or condensed ring. The above-described arylene group has a carbon atom number of usually 6 to 60, preferably 6 to 48, more preferably 6 to 30, further preferably 6 to 18. This carbon atom number does not include the carbon atom number of the substituent. The arylene group includes unsubstituted or substituted phenylene groups such as a 1,4-phenylene group, 1,3-phenylene group, 1,2-phenylene group and the like; unsubstituted or substituted naphthalenediyl groups such as a 1,4- naphthalenediyl group, 1,5- naphthalenediyl group, 2,6- naphthalenediyl group and the like; unsubstituted or substituted anthracenediyl groups such as a 1,4-anthracenediyl group, 1,5-anthracenediyl group, 2,6-anthracenediyl group, 9,10-anthracenediyl group and the like; unsubstituted or substituted phenanthrenediyl groups such as a 2,7-phenanthrenediyl group and the like; unsubstituted or substituted naphthacenediyl groups such as a 1,7-naphthacenediyl group, 2,8-naphthacenediyl group, 5,12-naphthacenediyl group and the like; unsubstituted or substituted fluorenediyl groups such as a 2,7-fluorenediyl group, 3,6-fluorenediyl group and the like; unsubstituted or substituted pyrenediyl groups such as a 1,6-pyrenediyl group, 1,8-pyrenediyl group, 2,7-pyrenediyl group, 4,9-pyrenediyl group and the like; and unsubstituted or substituted perylenediyl groups such as a 3,9-perylenediyl group, 3,10-perylenediyl group and the like; etc., and preferable are unsubstituted or substituted phenylene groups, and unsubstituted or substituted fluorenediyl groups.

The di-valent heterocyclic group indicates an atomic group remaining after removing two hydrogen atoms from a heterocyclic compound (particularly, aromatic heterocyclic compound), and means an unsubstituted di-valent heterocyclic group and a di-valent heterocyclic group substistuted by a substituent such as an alkyl group or the like. The di-valent heterocyclic group has a carbon atom number of usually 4 to 60, preferably 4 to 30, particularly preferably 6 to 12, not including the carbon atom number of the substituent. The above-described di-valent heterocyclic group includes unsubstituted or substituted pyridinediyl groups such as a 2,5-pyridinediyl group, 2,6-pyridinediyl group and the like; unsubstituted or substituted thiophenediyl groups such as a 2,5-thiophenediyl group and the like; unsubstituted or substituted furanediyl groups such as a 2,5-furanediyl group and the like; unsubstituted or substituted quinolinediyl groups such as a 2,6-quinolinediyl group and the like; unsubstituted or substituted isoquinolinediyl groups such as a 1,4-isoquinolinediyl group, 1,5-isoquinolinediyl group and the like; unsubstituted or substituted quinoxalinediyl groups such as a 5,8-quinoxalinediyl group and the like; unsubstituted or substituted benzo[1,2,5]thiadiazolediyl groups such as a 4,7-benzo[1,2,5]thiadiazolediyl group and the like; unsubstituted or substituted benzothiazolediyl groups such as a 4,7-benzothiazolediyl group and the like; unsubstituted or substituted carbazolediyl groups such as a 2,7-carbazolediyl group, 3,6-carbazolediyl group and the like; unsubstituted or substituted phenoxazinediyl groups such as a 3,7-phenoxazinediyl group and the like; unsubstituted or substituted phenothiazinediyl groups such as a 3,7-phenothiazinediyl group and the like; and unsubstituted or substituted dibenzosilolediyl groups such as a 2,7-dibenzosilolediyl group and the like; etc., and preferable are unsubstituted or substituted benzo[1,2,5]thiadiazolediyl groups, unsubstituted or substituted phenoxazinediyl groups, and unsubstituted or substituted phenothiazinediyl groups.

The di-valent group having a metal complex structure means an atomic group remaining after removing two hydrogen atoms from an organic ligand of a metal complex having the organic ligand and a center metal. The organic ligand has a carbon atom number of usually 4 to 60. The above-described organic ligand includes 8-quinolinol and derivatives thereof, benzoquinolinol and derivatives thereof, 2-phenylpyridine and derivatives thereof, 2-phenylbenzothiazole and derivatives thereof, 2-phenylbenzoxazole and derivatives thereof, porphyrin and derivatives thereof, and the like.

Examples of the center metal of the above-described metal complex include aluminum, zinc, beryllium, iridium, platinum, gold, europium, terbium and the like.

The above-described metal complex includes metal complexes known as low molecular weight fluorescence emitting materials and phosphorescence emitting materials, and triplet light emitting complexes, and the like.

### <Compound represented by the formula (1), or compound having residue of compound represented by the formula (1)>

In the above-described formula (1), when the aryl group optionally having a substituent or mono-valent heterocyclic group optionally having a substituent represented by Ar has a substituent, this substituent includes an alkyl group, alkoxy group, alkylthio group, aryl group, aryloxy group, arylthio group, arylalkyl group, arylalkoxy group, arylalkenyl group, arylalkynyl group, alkenyl group, alkynyl group, amino group, substituted amino group, silyl group, halogen atom, acyl group, acyloxy group, mono-valent heterocyclic group, heterocyclic thio group, imine residue, amide group, acid imide group, carboxyl group, nitro group, cyano group and the like. One or some or all of the hydrogen atoms contained in these substituents may be substituted by a fluorine atom.

In the above-described formula (1), Ar represents preferably a phenyl group, C₁ to C₁₂ alkoxyphenyl group, C₁ to C₁₂ alkylphenyl group, biphenyl group, C₁ to C₁₂ alkoxybiphenyl group, C₁ to C₁₂ alkylbiphenyl group, pyridylphenyl group or phenylpyridyl group, more preferably a phenyl group, C₁ to C₁₂ alkylphenyl group (for example, a phenyl group substituted by an alkyl group having 1 to 12 carbon atoms) or C₁ to C₁₂ alkylbiphenyl group (for example, a biphenyl group substituted by an alkyl group having 1 to 12 carbon atoms). These substituents optionally have a substituent.

As the compound represented by the above-described formula (1), preferable are compounds represented by the following formulae (1)' and (1)'' from the standpoint of injection and transportation of electrons. 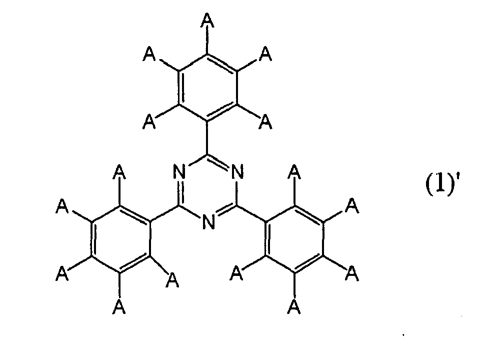 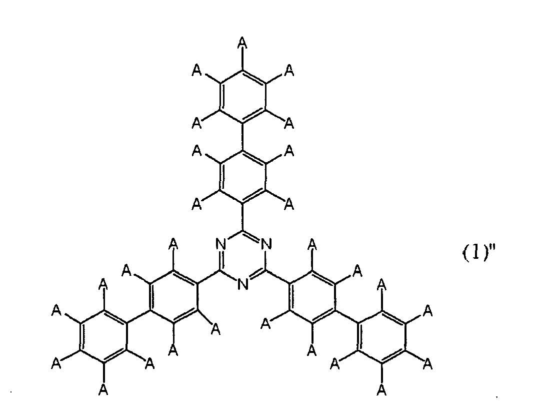 wherein A represents a hydrogen atom, an alkyl group, an alkoxy group, an alkylthio group, an aryl group, an aryloxy group, an arylthio group, an arylalkyl group, an arylalkoxy group, an arylalkenyl group, an arylalkynyl group, an alkenyl group, an alkynyl group, an amino group, a substituted amino group, a silyl group, a halogen atom, an acyl group, an acyloxy group, a mono-valent heterocyclic group, a heterocyclic thio group, an imine residue, an amide group, an acid imide group, a carboxyl group, a nitro group, or a cyano group; one or some or all of the hydrogen atoms contained in groups represented by A may be substituted by a fluorine atom. A plurality of As may be the same or different.

As the compound represented by the above-described formula (1), the following compounds are also mentioned.

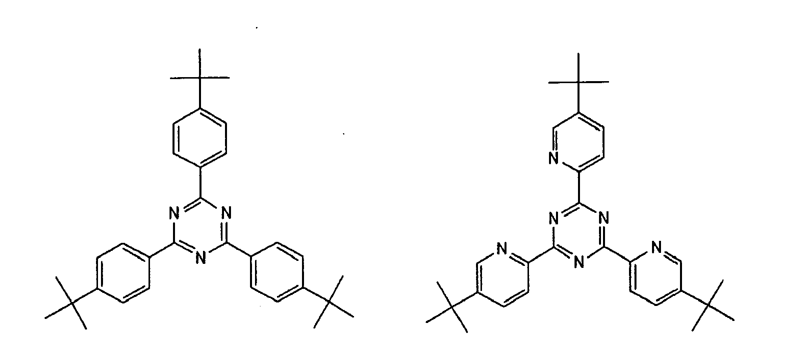

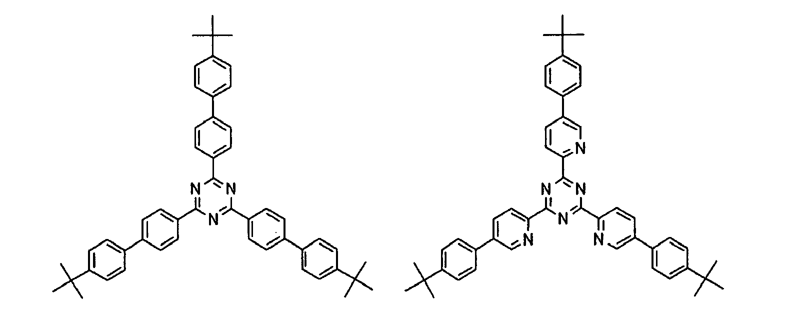

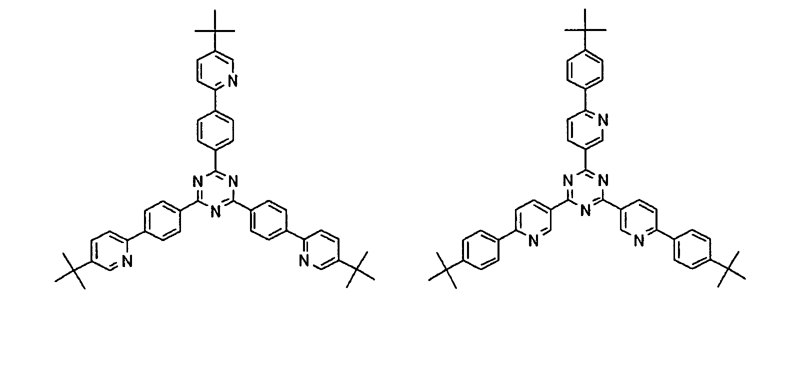

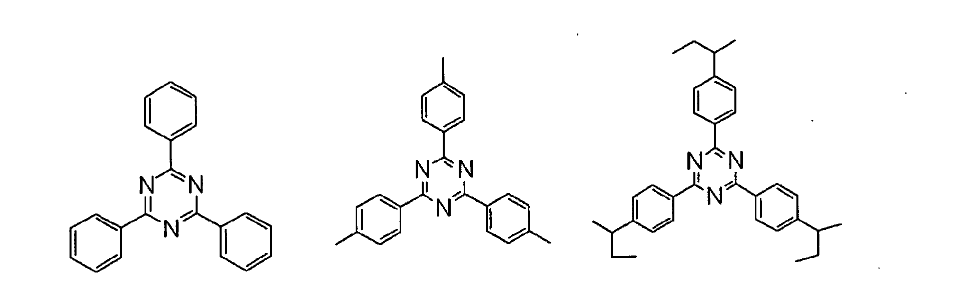

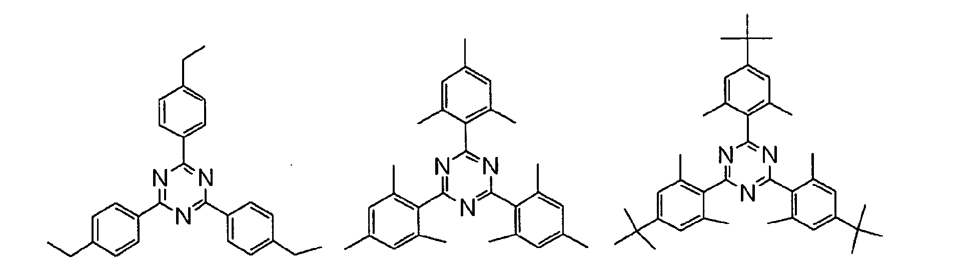

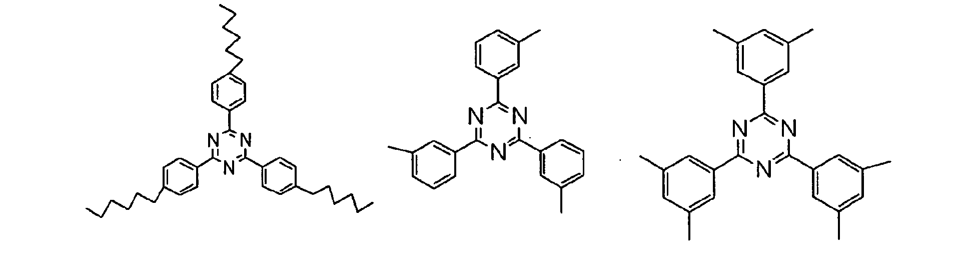

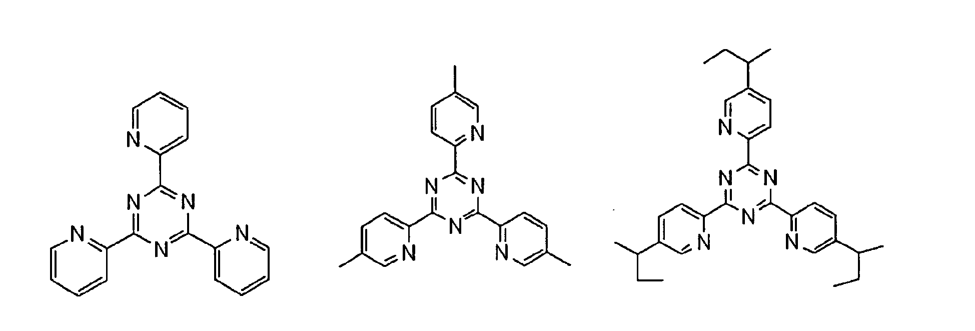

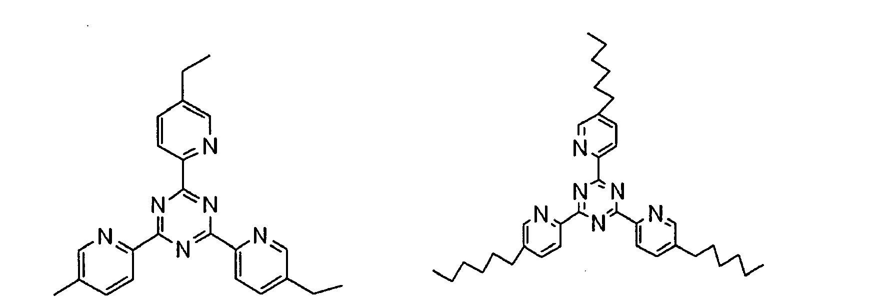

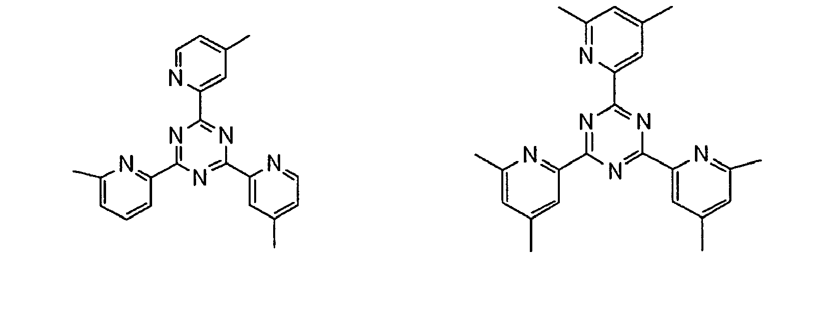

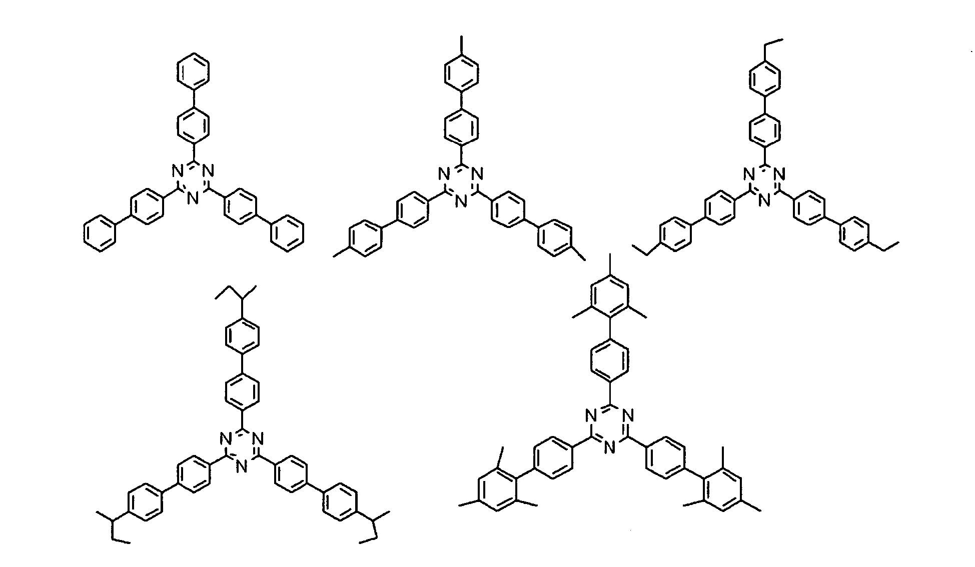

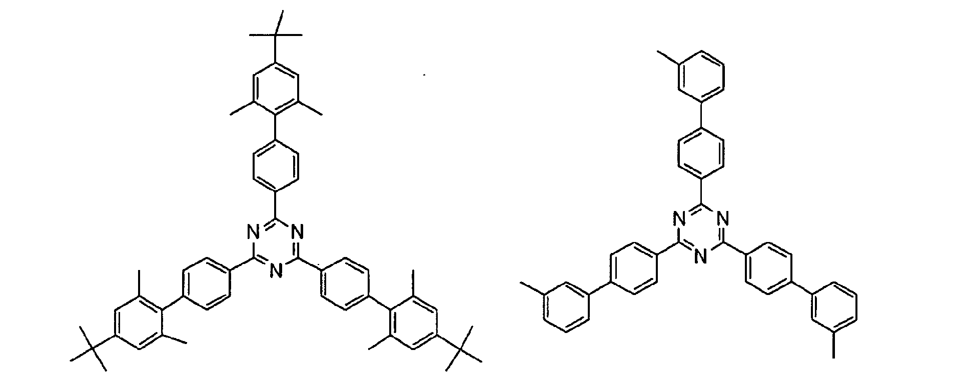

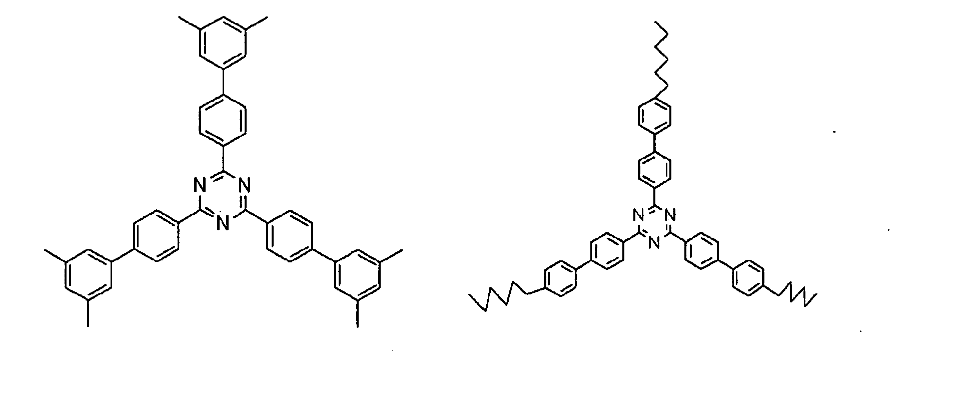

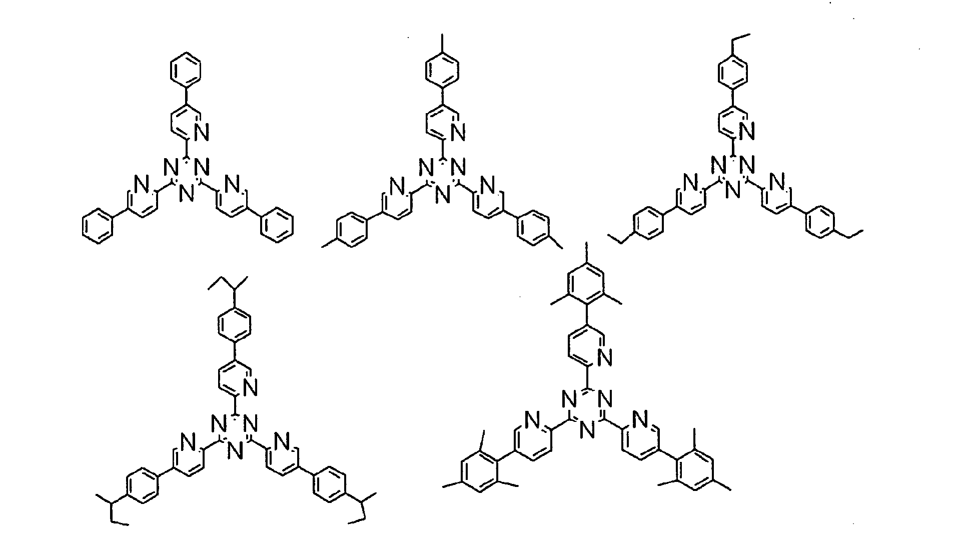

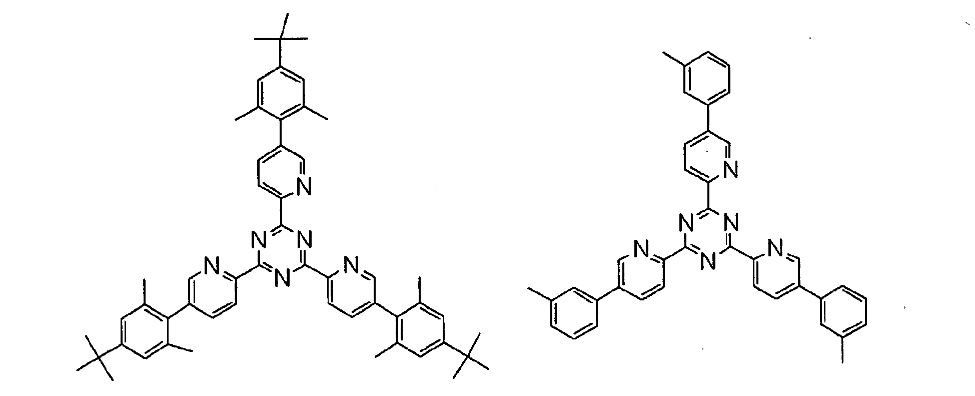

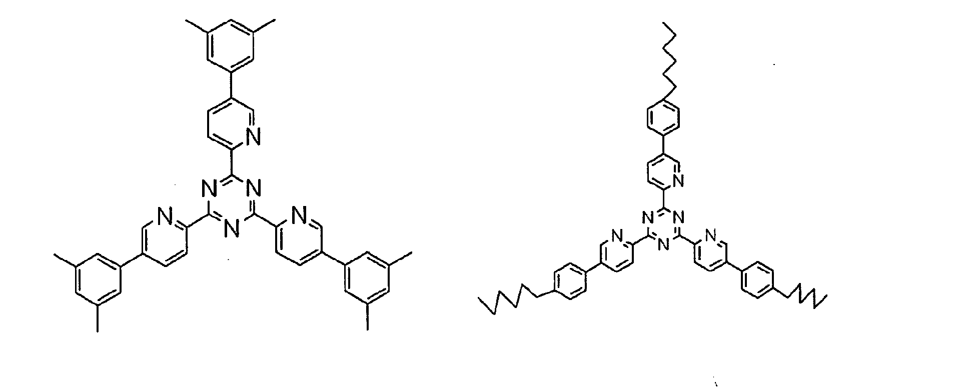

The compound having a residue of a compound represented by the above-described formula (1) is a compound having a residue which is an atomic group remaining after removing a hydrogen atom of an aryl group optionally having a substituent or a mono-valent heterocyclic group optionally having a substituent represented by Ar in the above-described formula (1).

The compound having a residue of a compound represented by the above-described formula (1) may be a low molecular weight compound or a polymer compound, and examples thereof include polymer compounds having as a repeating unit a residue which is an atomic group obtained by removing one hydrogen atom and polymer compounds having as a repeating unit a residue which is an atomic group obtained by removing two hydrogen atom.

The polymer compound has a polystyrene-equivalent number average molecular weight in the range of usually 10³ to 10⁷ and has a polystyrene-equivalent weight average molecular weight in the range of usually 10³ to 10⁸.

When the compound having a residue of a compound represented by the above-described formula (1) is a polymer compound, it is preferably a conjugated polymer from the standpoint of injection and transportation of charges. The conjugated polymer means a polymer compound in which 50 to 100%, particularly 70 to 100%, especially 90 to 100% of all bonds in the main chain are conjugated.

When the compound having a residue of a compound represented by the above-described formula (1) is a polymer compound, it is preferable that the polymer compound has at least one repeating unit represented by any of the following formulae (A), (B) and (C), from the standpoint of transportation and injection of charges and luminance half life. 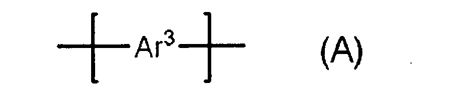 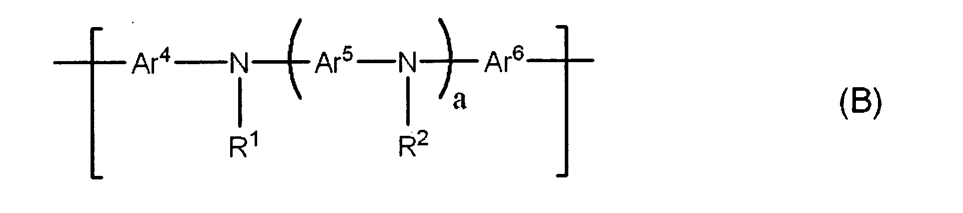 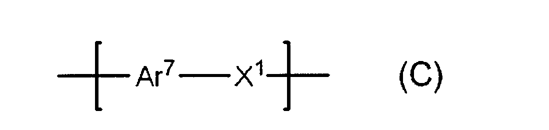 wherein Ar³ and Ar⁷ represent each independently an arylene group optionally having a substituent, a di-valent heterocyclic group optionally having a substituent, or di-valent group having a metal complex structure optionally having a substituent; Ar⁴, Ar⁵ and Ar⁶ represent each independently an arylene group optionally having a substituent, a di-valent heterocyclic group optionally having a substituent, or a di-valent group obtained by connection via a single bond of two aromatic rings optionally having a substituent; R¹ and R² represent each independently a hydrogen atom, an alkyl group, an aryl group, a mono-valent heterocyclic group or an arylalkyl group; X¹ represents -CR³=C^{R}4 -or C=C-; R³ and R⁴ represent each independently a hydrogen atom, an alkyl group, an aryl group, a mono-valent heterocyclic group, a carboxyl group, or a cyano group;a represents 0 or 1.

### -Repeating unit represented by the formula (A)-

In the above-described formula (A), when the group represented by Ar³ has a substituent, this substituent includes an alkyl group, alkoxy group, aryl group, aryloxy group, arylalkyl group, arylalkoxy group, arylalkenyl group, arylalkynyl group, amino group, substituted amino group, halogen atom, acyl group, acyloxy group, mono-valent heterocyclic group, carboxyl group, nitro group, cyano group and the like, preferably an alkyl group, alkoxy group, aryl group, aryloxy group, substituted amino group and mono-valent heterocyclic group, more preferably an alkyl group, alkoxy group and aryl group.

The arylene group in the arylene group optionally having a substituent represented by Ar³ in the above-described formula (A) means an atomic group obtained by removing two hydrogen atoms from an aromatic hydrocarbon, and includes those having an independent benzene ring or condensed ring. This arylene group has a carbon atom number of usually 6 to 60, preferably 6 to 30, more preferably 6 to 18. The arylene group in the arylene group optionally having a substituent represented by Ar³ in the above-described formula (A) includes a 1,4-phenylene group, 1,3-phenylene group, 1,4-naphthalenediyl group, 1,5-naphthalenediyl group, 2,6-naphthalenediyl group, 9,10-anthracenediyl group, 2,7-phenanthrylene group, 5,12-naphthacenylene group, 2,7-fluorenediyl group, 3,6-fluorenediyl group, 1,6-pyrenediyl group, 1,8-pyrenediyl group, 3,9-perylenediyl group, 3,10-perylenediyl group, 2,6-quinolinediyl group, 1,4-isoquinolinediyl group, 1,5-isoquinolinediyl group, 5,8-quinoxalinediyl group and the like, preferably a 1,4-phenylene group, 1,4-naphthalenediyl group, 1,5-naphthalenediyl group, 2,6-naphthalenediyl group, 9,10-anthracenediyl group, 2,7-fluorenediyl group, 1,6-pyrenediyl group, 3,9-perylenediyl group, 3,10-perylenediyl group, 2,6-quinolinediyl group, 1,4-isoquinolinediyl group and 5,8-quinoxalinediyl group, more preferably a 1,4-phenylene group, 1,4-naphthalenediyl group, 1,5-naphthalenediyl group, 2,6-naphthalenediyl group, 9,10-anthracenediyl group, 2,7-fluorenediyl group and 5,8-quinoxalinediyl group, particularly preferably a 1,4-phenylene group and 2,7-fluorenediyl group.

The di-valent heterocyclic group in the di-valent heterocyclic group optionally having a substituent represented by Ar³ in the above-described formula (A) includes a 4,7-benzo[1,2,5]thiadiazolediyl group, 3,7-phenoxazinediyl group, 3,7-phenothiazinediyl group and the like, preferably a 4,7-benzo[1,2,5]thiadiazolediyl group, 3,7-phenoxazinediyl group and 3,7-phenothiazinediyl group, more preferably a 4,7-benzo[1,2,5]thiadiazolediyl group, 3,7-phenoxazinediyl group and 3,7-phenothiazinediyl group, particularly preferably a 4,7-benzo[1,2,5]thiadiazolediyl group, 3,7-phenoxazinediyl group and 3,7-phenothiazinediyl group.

The di-valent group having a metal complex structure optionally having a substituent represented by Ar³ in the above-described formula (A) includes, for example, groups represented by the following formulae M-1 to M-7.

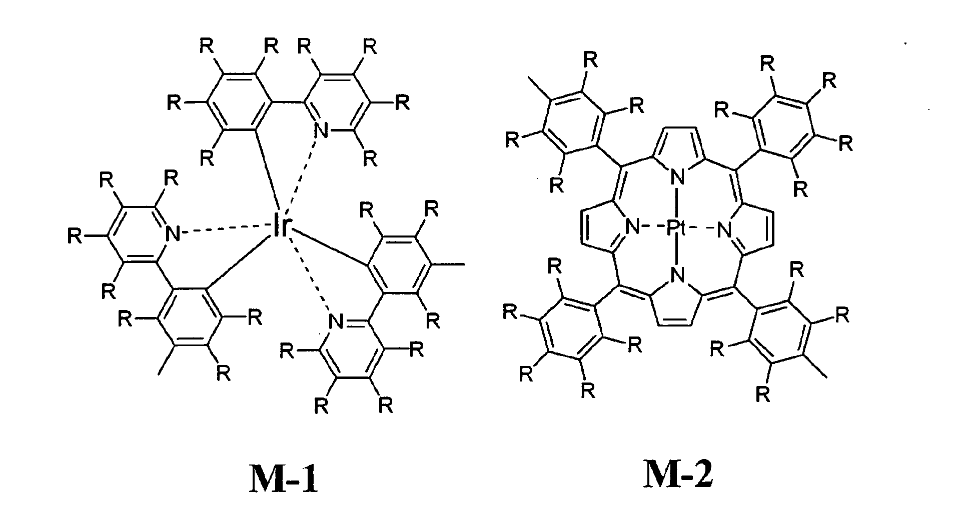

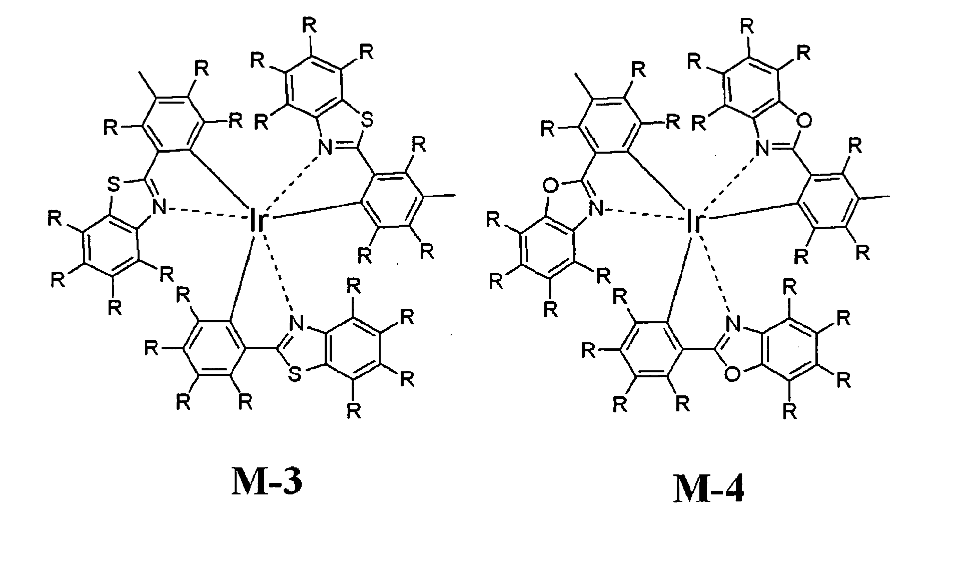

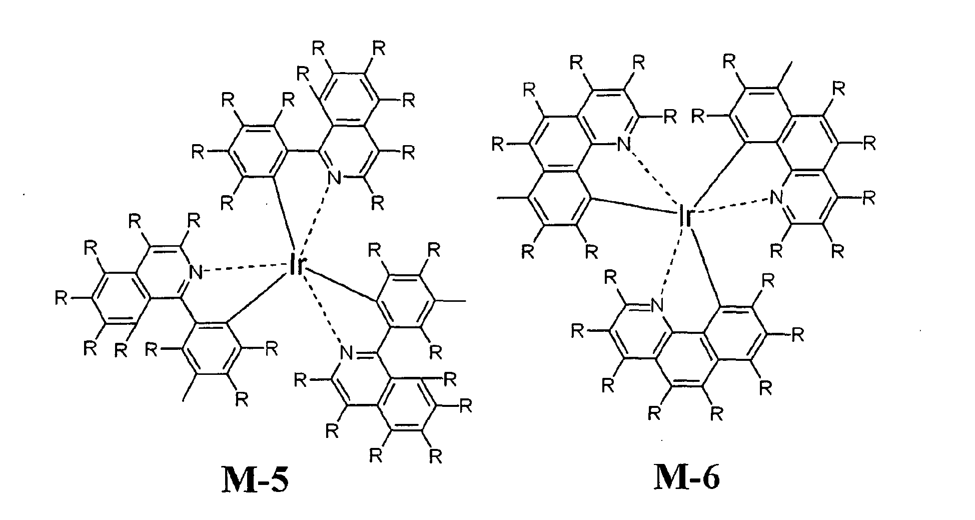

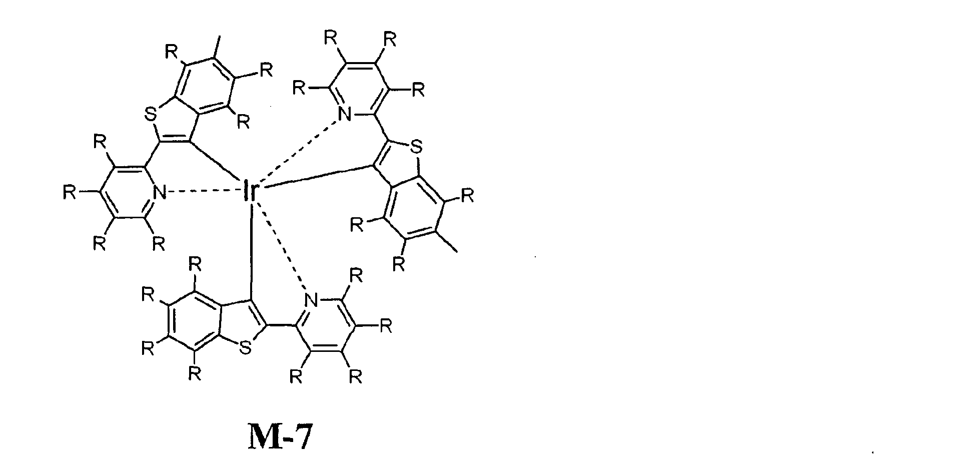

Among them, the group represented by Ar³ is desirably at least one group represented by the following formulae (D), (E), (F), (G) and (H). 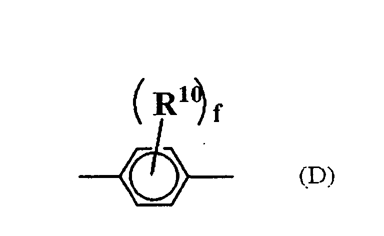 wherein R¹⁰ represents an alkyl group, an alkoxy group, an aryl group, an aryloxy group, an arylalkyl group, an arylalkoxy group, an arylalkenyl group, an arylalkynyl group, an amino group, a substituted amino group, a halogen atom, an acyl group, an acyloxy group, a mono-valent heterocyclic group, a carboxyl group, a nitro group or a cyano group; one or some of all of the hydrogen atoms contained in these groups may be substituted by a fluorine atom; f represents an integer of 0 to 4; when a plurality of R¹⁰s exist, they may be the same or different. 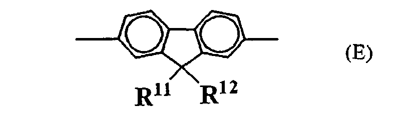 wherein R¹¹ and R¹² represent each independently a hydrogen atom, an alkyl group, an aryl group, an arylalkyl group or
a mono-valent heterocyclic group. 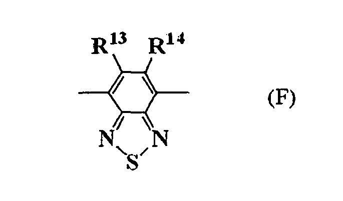 wherein R¹³ and R¹⁴ represent each independently a hydrogen atom, an alkyl group, an alkoxy group, an aryl group, an aryloxy group, an arylalkyl group, an arylalkoxy group, an arylalkenyl group, an arylalkynyl group, an amino group, a substituted amino group, a halogen atom, an acyl group, an acyloxy group, a mono-valent heterocyclic group, carboxyl group, a nitro group or a cyano group; one or some or all of the hydrogen atoms contained in these groups may be substituted by a fluorine atom. 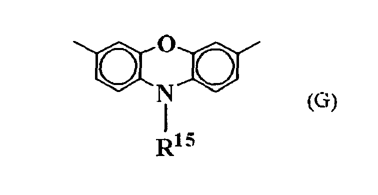 wherein R¹⁵ represents a hydrogen atom, an alkyl group, an aryl group, a mono-valent heterocyclic group or an arylalkyl group. 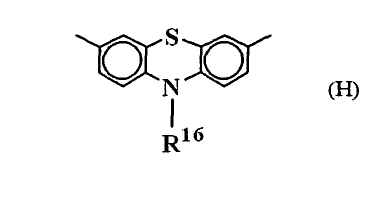 wherein R¹⁶ represents a hydrogen atom, an alkyl group, an aryl group, a mono-valent heterocyclic group or an arylalkyl group.

In the above-described formula (D), R¹⁰ represents preferably an alkyl group, alkoxy group, aryl group, aryloxy group, arylalkyl group, arylalkoxy group, arylalkenyl group, arylalkynyl group, substituted amino group, acyl group or mono-valent heterocyclic group, more preferably an alkyl group, alkoxy group, aryl group, aryloxy group, substituted amino group, acyl group or mono-valent heterocyclic group, further preferably an alkyl group, alkoxy group, aryl group or mono-valent heterocyclic group, particularly preferably an alkyl group, alkoxy group or aryl group.

In the above-described formula (D), f represents preferably an integer of 0 to 2.

In the above-described formula (E), R¹¹ and R¹² represent preferably an alkyl group, aryl group or mono-valent heterocyclic group, more preferably an alkyl group or aryl group.

In the above-described formula (F), R¹³ and R¹⁴ represent preferably a hydrogen atom, alkyl group, alkoxy group, aryl group, aryloxy group, arylalkyl group, arylalkoxy group, substituted amino group, acyl group or mono-valent heterocyclic group, more preferably a hydrogen atom, alkyl group, alkoxy group, aryl group, aryloxy group or mono-valent heterocyclic group, further preferably a hydrogen atom or alkyl group, particularly preferably a hydrogen atom.

In the above-described formula (G), R¹⁵ represents preferably an alkyl group, aryl group or mono-valent heterocyclic group, more preferably an alkyl group or aryl group, further preferably an aryl group.

In the above-described formula (H), R¹⁶ represents preferably an alkyl group, aryl group or mono-valent heterocyclic group, more preferably an alkyl group or aryl group, further preferably an aryl group.

### -Repeating unit represented by the formula (B)-

In the above-described formula (B), when the group represented by Ar⁴, Ar⁵ and Ar⁶ has a substituent, this substituent includes, for example, an alkyl group, alkoxy group, aryl group, aryloxy group, arylalkyl group, arylalkoxy group, arylalkenyl group, arylalkynyl group, amino group, substituted amino group, halogen atom, acyl group, acyloxy group, mono-valent heterocyclic group, carboxyl group, nitro group and cyano group, preferably an alkyl group, alkoxy group, aryl group, aryloxy group, arylalkyl group, arylalkoxy group, substituted amino group, acyl group and cyano group, more preferably an alkyl group, alkoxy group and aryl group.

The arylene group in the arylene group optionally having a substituent represented by Ar⁴, Ar⁵ and Ar⁶ in the above-described formula (B) means an atomic group obtained by removing two hydrogen atoms from an aromatic hydrocarbon, and includes those having an independent benzene ring or condensed ring. This arylene group has a carbon atom number of usually 6 to 60, preferably 6 to 30, more preferably 6 to 18.

The arylene group in the arylene group optionally having a substituent represented by Ar⁴, Ar⁵ and Ar⁶ in the above-described formula (B) includes a 1,3-phenylene group, 1,4-phenylene group, 1,4-naphthalenediyl group, 2,6-naphthalenediyl group, 9,10-anthracenediyl group, 2,7-phenanthrenediyl group, 5,12-naphthacenediyl group, 2,7-fluorenediyl group, 3,8-perylenediyl group and the like.

The di-valent heterocyclic group in the di-valent heterocyclic group optionally having a substituent represented by Ar⁴, Ar⁵ and Ar⁶ in the above-described formula (B) has a carbon atom number of usually 4 to 60, preferably 4 to 20, more preferably 4 to 9. The di-valent heterocyclic group in the di-valent heterocyclic group optionally having a substituent represented by Ar⁴, Ar⁵ and Ar⁶ in the above-described formula (B) includes a 2,5-thiophenediyl group, N-methyl-2,5-pyrrolediyl group, 2,5-furanediyl group, 4,7-benzo[1,2,5]thiadiazolediyl group, 3,7-phenoxazinediyl group, 3,6-carbazolediyl group and the like.

The di-valent group obtained by connection via a single bond of two aromatic rings in the di-valent group obtained by connection via a single bond of two aromatic rings optionally has a substituent represented by Ar⁴, Ar⁵ and Ar⁶ in the above-described formula (A) includes, for example, groups represented by the following formulae (3A-1) to (3A-4).

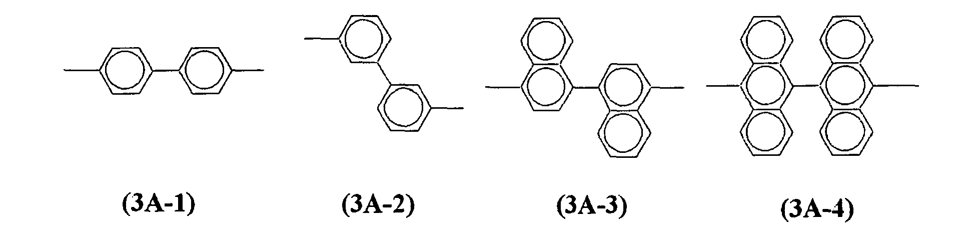

In the above-described formula (B), Ar⁴ and Ar⁶ represent each independently preferably an arylene group optionally having a substituent, more preferably a 1,3-phenylene group optionally having a substituent, 1,4-phenylene group optionally optionally having a substituent, 1,4-naphthalenediyl group optionally having a substituent, 2,6-naphthalenediyl group optionally having a substituent or group represented by the above-described formula (3A-1), more preferably, a 1,4-phenylene group optionally having a substituent or 1,4-naphthalenediyl group optionally having a substituent, particularly preferably, a 1,4-phenylene group optionally having a substituent.

In the above-described formula (B), Ar⁵ represents preferably a 1,3-phenylene group optionally having a substituent, 1,4-phenylene group optionally having a substituent, 1,4-naphthalenediyl group optionally having a substituent, 2,7-fluorenediyl group optionally having a substituent, 4,7-benzo[1,2,5]thiadiazolediyl group optionally having a substituent, 3,7-phenoxazinediyl group optionally having a substituent, group represented by the above-described formula (3A-1) or group represented by the above-described formula (3A-4), preferably a 1,4-phenylene group optionally having a substituent, 1,4-naphthalenediyl group optionally having a substituent, 2,7-fluorenediyl group optionally having a substituent or group represented by the above-described formula (3A-1), further preferably a 1,4-phenylene group optionally having a substituent or group represented by the above-described formula (3A-1) optionally having a substituent.

In the above-described formula (B), R¹ and R² represent each independently preferably an alkyl group, aryl group or mono-valent heterocyclic group, more preferably an alkyl group or aryl group, further preferably an aryl group.

The repeating unit represented by the above-described formula (B) includes repeating units represented by the following formulae (3B-1) to (3B-4). In the formulae, R^{a} represents a hydrogen atom, alkyl group, alkoxy group, aryl group, aryloxy group, arylalkyl group, arylalkoxy group, arylalkenyl group, arylalkynyl group, amino group, substituted amino group, halogen atom, acyl group, acyloxy group, mono-valent heterocyclic group, carboxyl group, nitro group or cyano group. A plurality of R^{a}s may be the same or different.

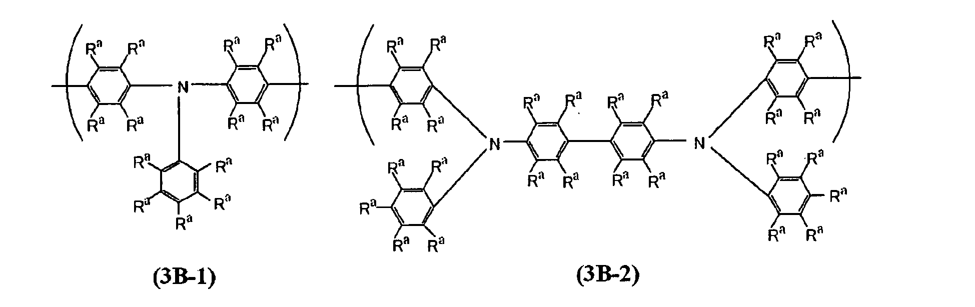

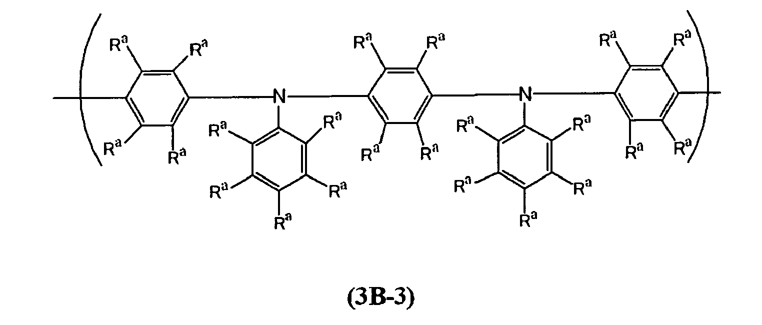

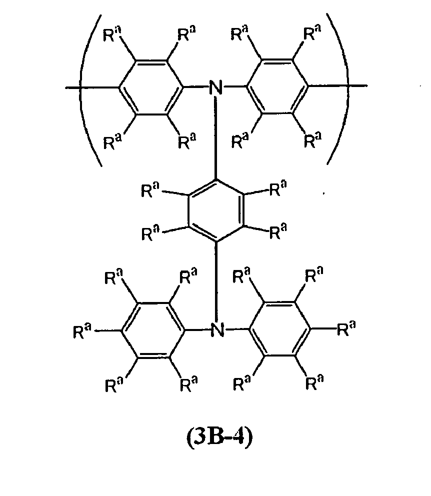

### -Repeating unit represented by the formula (C)-

The arylene group optionally having a substituent, di-valent heterocyclic group optionally having a substituent and di-valent group having a metal complex structure optionally having a substituent represented by Ar⁷ in the above-described formula (C) are the same as those explained and exemplified in the above-described section of Ar³.

In the above-described formula (C), R³ and R⁴ represent preferably a hydrogen atom, alkyl group or aryl group, more preferably a hydrogen atom or aryl group.

The repeating unit represented by the above-described formula (C) includes, for example, repeating units represented by the following formulae (4A-1) to (4A-11).

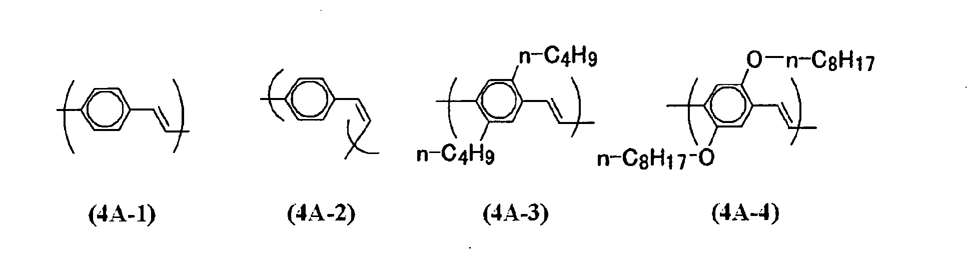

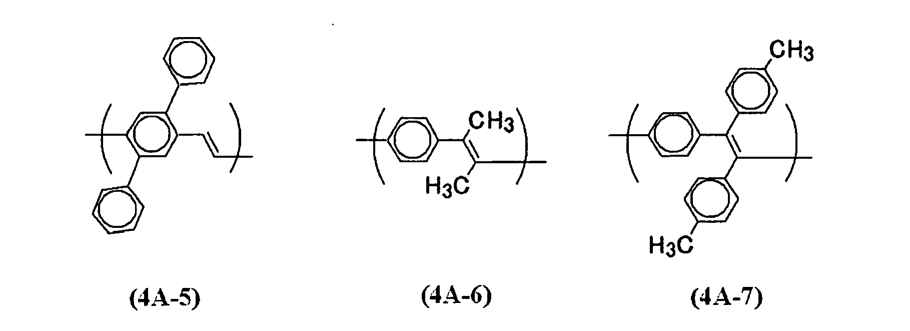

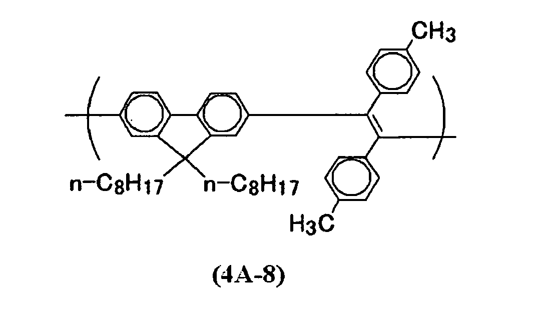

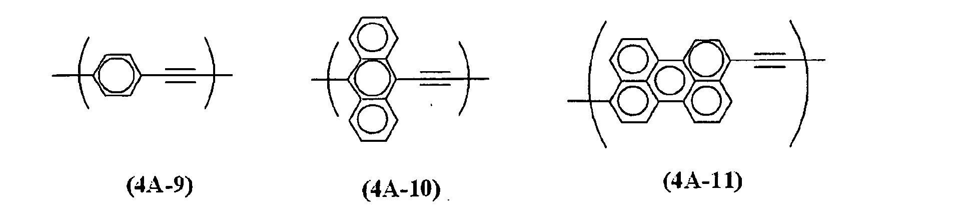

The polymer compound having as a repeating unit a residue which is an atomic group obtained by removing two hydrogen atoms in an aryl group optionally having a substituent or mono-valent heterocyclic group optionally having a substituent represented by Ar in the above-described formula (1) includes, for example, polymer compounds having a repeating unit represented by the following formula (3). 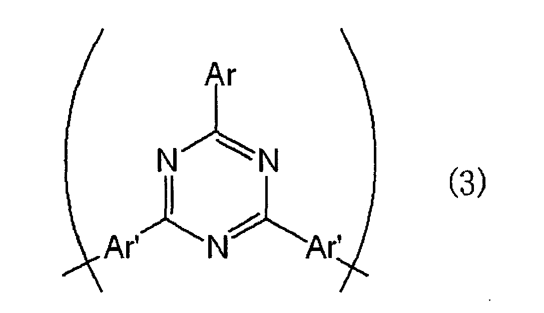 wherein Ar has the same meaning as described above; each Ar' represents an arylene group optionally having a substituent or a di-valent heterocyclic group optionally having a substituent.

In the above-described formula (3), when the aryl group optionally having a substituent or mono-valent heterocyclic group optionally having a substituent represented by Ar has a substituent, or when the arylene group optionally having a substituent or di-valent heterocyclic group optionally having a substituent represented by Ar' has a substituent, the substituent includes an alkyl group, alkoxy group, alkylthio group, aryl group, aryloxy group, arylthio group, arylalkyl group, arylalkoxy group, arylalkenyl group, arylalkynyl group, alkenyl group, alkynyl group, amino group, substituted amino group, silyl group, halogen atom, acyl group, acyloxy group, mono-valent heterocyclic group, heterocyclic thio group, imine residue, amide group, acid imide group, carboxyl group, nitro group, cyano group and the like. One or some or all of the hydrogen atoms contained in these substituents may be substituted by a fluorine atom.

Ar' represents, for example, a phenylene group, C₁ to C₁₂ alkoxyphenylene group, C₁ to C₁₂ alkylphenylene group, biphenylene group, C₁ to C₁₂ alkoxybiphenylene group, C₁ to C₁₂ alkylbiphenylene group, pyridinediyl group, C₁ to C₁₂ alkoxypyridinediyl group or C₁ to C₁₂ alkylpyridinediyl group, preferably, a 1,4-phenylene group, 1,3-phenylene group, 1,2-phenylene group, 1,4-pyridinediyl group, 1,3-pyridinediyl group, 1,2-pyridinediyl group, 1,4-naphthalenediyl group, 2,6-naphthalenediyl group, 1,4-anthracenediyl group, 1,5-anthracenediyl group, 2,6-anthracenediyl group or 9,10-anthracenediyl group, more preferably, a 1,4-phenylene group, 1,3-phenylene group, 1,2-phenylene group, 1,4-pyridinediyl group, 1,3-pyridinediyl group, 1,2-pyridinediyl group, 1,4-naphthalenediyl group or 2,6-naphthalenediyl group, further preferably, a 1,4-phenylene group, 1,3-phenylene group, 1,4-naphthalenediyl group, 1,4-pyridinediyl group, 1,3-pyridinediyl group or 1,2-pyridinediyl group, particularly preferably, a 1,4-phenylene group or 1,4-pyridinediyl group.

As the polymer compound having a repeating unit represented by the above-described formula (3), preferable are polymer compounds in which Ar represents a phenyl group and Ar' represents a 1,4-phenylene group in the above-described formula from the standpoint of injection and transportation of charges.

As the polymer compound having a repeating unit represented by the above-described formula (3), preferable are compounds represented by the following formulae (3)' and (3)'' from the standpoint of injection and transportatio of electrons. 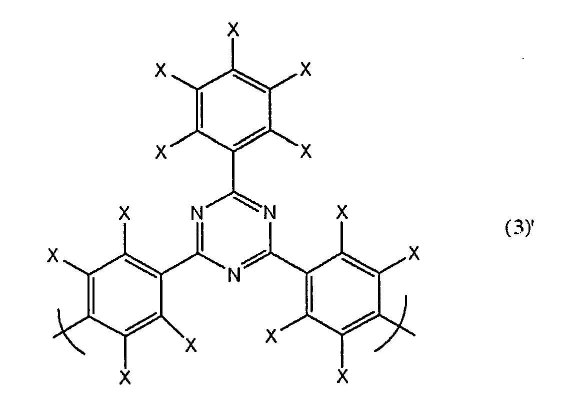 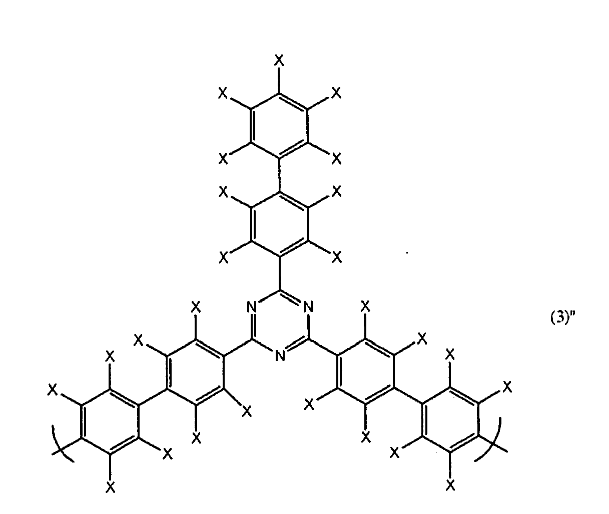 wherein X represents a hydrogen atom, an alkyl group, an alkoxy group, an alkylthio group, an aryl group, an aryloxy group, an arylthio group, an arylalkyl group, an arylalkoxy group, an arylalkenyl group, an arylalkynyl group, an alkenyl group, an alkynyl group, an amino group, a substituted amino group, a silyl group, a halogen atom, an acyl group, an acyloxy group, a mono-valent heterocyclic group, a heterocyclic thio group, an imine residue, an amide group, an acid imide group, a carboxyl group, a nitro group or a cyano group; one or some or all of the hydrogen atoms contained in the group represented by X may be substituted by a fluorine atom; a plurality of Xs may be the same or different.

The polymer compound having a repeating unit represented by the above-described formula (3) includes also polymer compounds having one or both of repeating units represented by the following formulae.

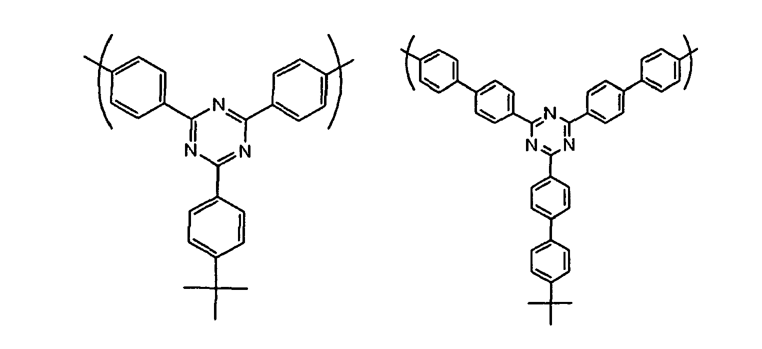

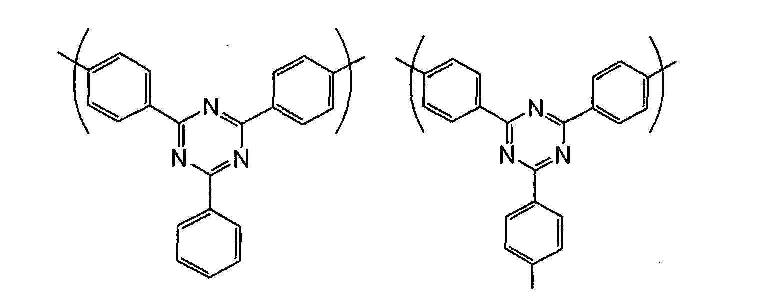

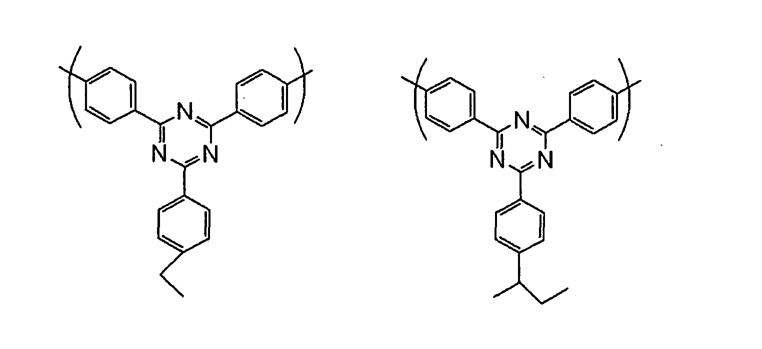

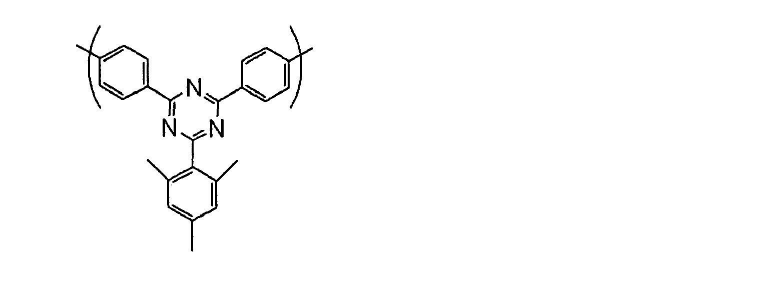

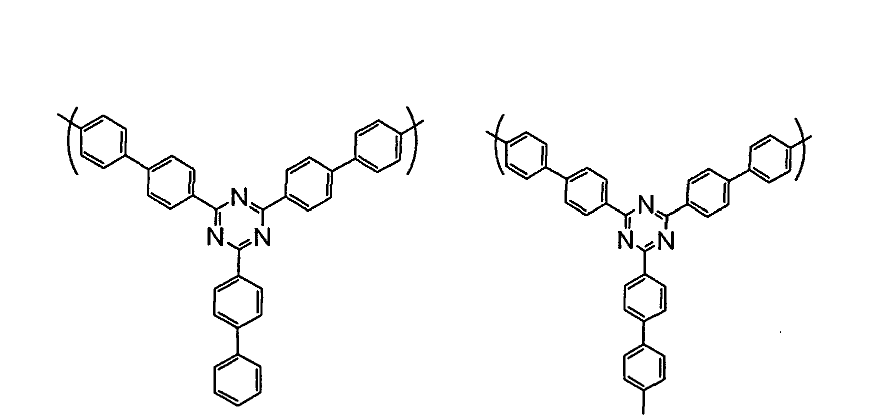

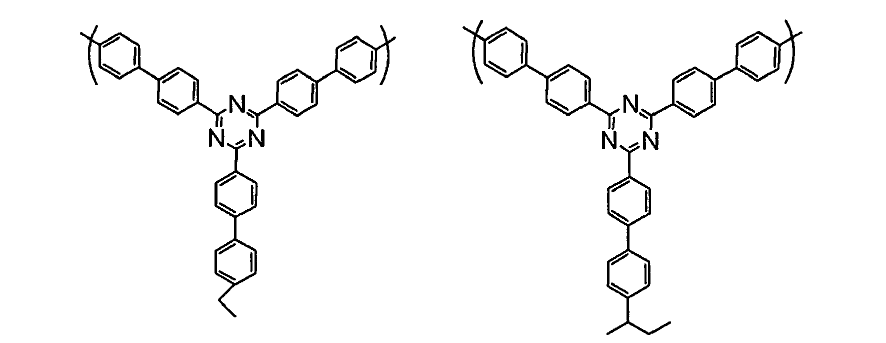

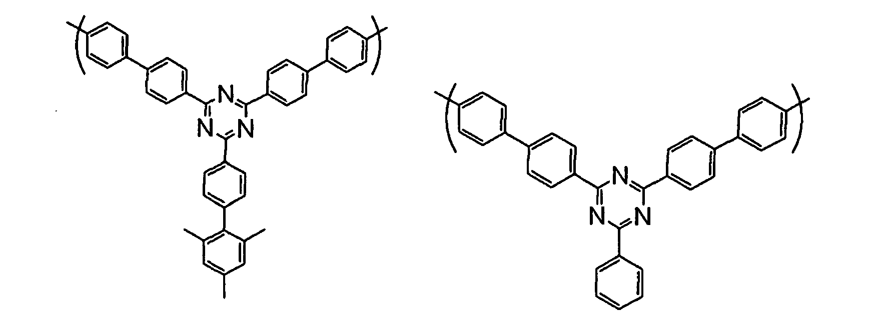

The compounds represented by the above-described formula (1) and the compounds having residues of compounds represented by the above-described formula (1) may be used singly or in combination.

When the compound having a residue of a compound represented by the above-described formula (1) is a compound having at least one of repeating units represented by the above-described formulae (A) to (C), the sum of molar numbers of the residue of a compound represented by the above-described formula (1), the repeating unit represented by the above-described formula (A), the repeating unit represented by the above-described formula (B) and the repeating unit represented by the above-described formula (C) is preferably 50 to 100 mol%, more preferably 70 to 100 mol%, particularly preferably 80 to 100 mol%, with respect to the sum of molar numbers of all repeating units contained in the polymer compound.

### <Compound represented by the formula (2) or compound having residue of compound represented by the formula (2)>

The composition of the present invention contains a compound represented by the above-described formula (2) or compound having a residue of a compound represented by the above-described formula (2), together with the compound represented by the above-described formula (1) or compound having a residue of a compound represented by the above-described formula (1).

In -C(R')= represented by 8 members among Z¹, Z², Z³, Z⁹, Z⁵, Z⁶, Z⁷, Z⁸, Z⁹ and Z¹⁰ in the above-described formula (2), R' represents a hydrogen atom, alkyl group optionally having a substituent, alkoxy group optionally having a substituent, alkylthio group optionally having a substituent, aryl group optionally having a substituent, aryloxy group optionally having a substituent, arylthio group optionally having a substituent, alkenyl group optionally having a substituent, alkynyl group optionally having a substituent, amino group optionally having a substituent, silyl group optionally having a substituent, halogen atom, acyl group optionally having a substituent, acyloxy group optionally having a substituent, mono-valent heterocyclic group optionally having a substituent, heterocyclic thio group optionally having a substituent, imine residue, amide group optionally having a substituent, acid imide group, carboxyl group, nitro group or cyano group, preferably, a hydrogen atom, alkyl group optionally substituted by a fluorine atom, alkoxy group optionally substituted by a fluorine atom, aryl group optionally substituted by a fluorine atom, aryloxy group optionally substituted by a fluorine atom, arylalkyl group optionally substituted by a fluorine atom, arylalkoxy group optionally substituted by a fluorine atom, arylalkenyl group optionally substituted by a fluorine atom, arylalkynyl group optionally substituted by a fluorine atom, amino group optionally substituted by a fluorine atom, substituted amino group optionally substituted by a fluorine atom, halogen atom, acyl group optionally substituted by a fluorine atom, acyloxy group optionally substituted by a fluorine atom, mono-valent heterocyclic group optionally substituted by a fluorine atom, carboxyl group, nitro group or cyano group, further preferably, a hydrogen atom, alkyl group optionally substituted by a fluorine atom, alkoxy group optionally substituted by a fluorine atom, aryl group optionally substituted by a fluorine atom, aryloxy group optionally substituted by a fluorine atom, arylalkyl group optionally substituted by a fluorine atom, arylalkoxy group optionally substituted by a fluorine atom, halogen atom or mono-valent heterocyclic group optionally substituted by a fluorine atom, more preferably, a hydrogen atom, alkyl group optionally substituted by a fluorine atom, aryl group optionally substituted by a fluorine atom, arylalkyl group optionally substituted by a fluorine atom, halogen atom or mono-valent heterocyclic group optionally substituted by a fluorine atom, particularly preferably, a hydrogen atom, alkyl group optionally substituted by a fluorine atom or aryl group optionally substituted by a fluorine atom, especially preferably, a hydrogen atom or alkyl group optionally substituted by a fluorine atom.

It is preferable that the position of -N= represented by one member among Z¹, Z² and Z³ and the position of -N= represented by one member among Z⁶, Z⁷ and Z⁸ are symmetrical in the above-described formula (2). For example, a case in which Z¹ and Z⁶ represent -N= and Z²,

Z³ , Z⁷ and Z⁸ represent -C(R')=, a case in which Z² and Z⁷ represent -N= and Z¹, Z³, Z⁶ and Z⁸ represent -C(R')= and a case in which Z³ and Z⁸ represent -N= and Z¹, Z², Z⁶ and Z⁷ represent -C(R')= are preferable, and a case in which Z¹ and Z⁶ represent -N= and Z², Z³, Z⁷ and Z⁸ represent - C(R')= is more preferable.

The compound represented by the above-described formula (2) includes the following compounds, and preferable are 2,2'-bipyridyl, 5-methyl-2,2'-bipyridyl, 5,5'-dimethyl-2,2'-bipyridyl, 3,3'-bipyridyl and 4,4'-bipyridyl from the standpoint of the luminance half life of an organic electroluminescent device.

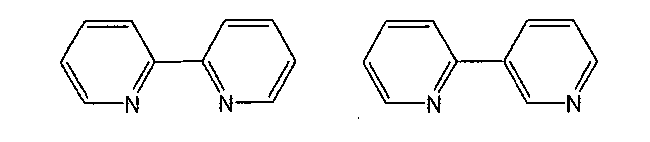

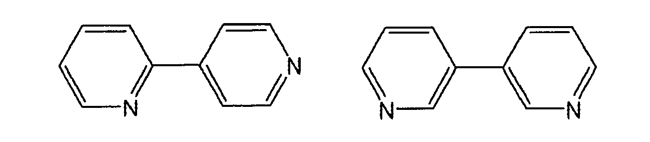

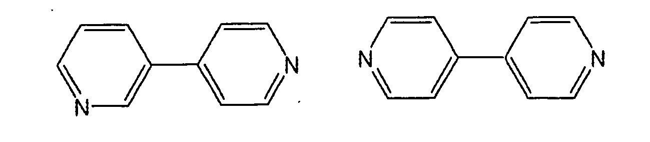

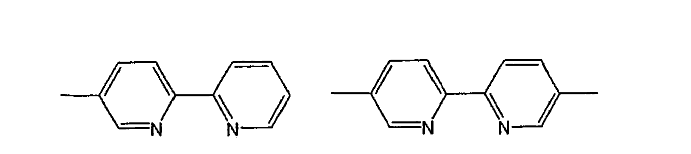

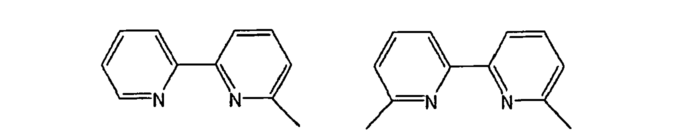

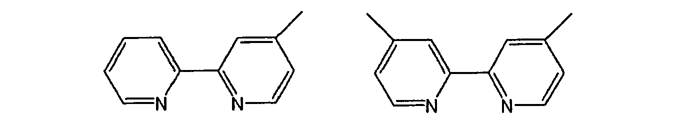

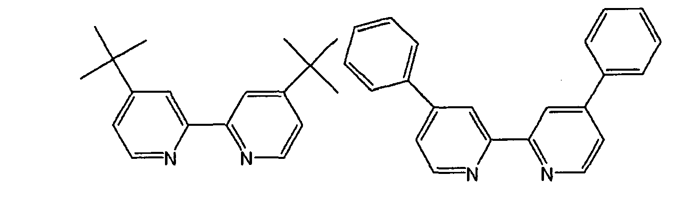

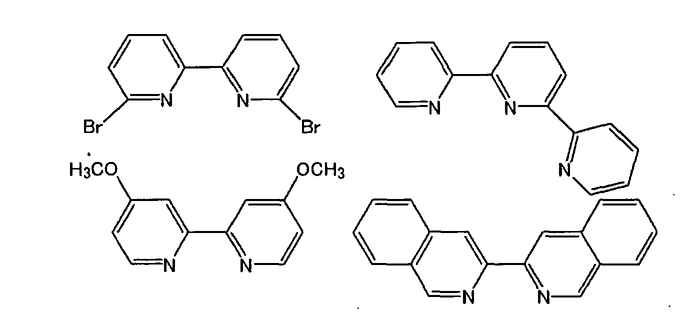

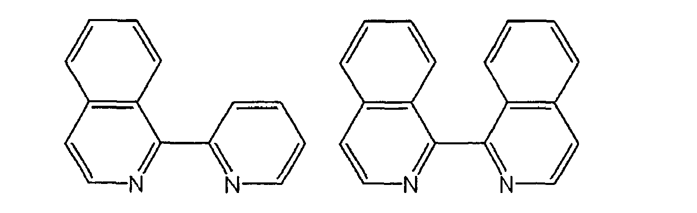

The compound having a residue of a compound represented by the above-described formula (2) is a compound having a residue which is an atomic group remaining after removing R' from a group represented by - C(R')= in the above-described formula (2).

The compound having a residue of a compound represented by the above-described formula (2) may be a low molecular weight compound or a polymer compound and includes, for example, polymer compounds having as a repeating unit a residue which is an atomic group obtained by removing one R' and polymer compounds having as a repeating unit a residue which is an atomic group obtained by removing two R's.

When the compound having a residue of a compound represented by the above-described formula (2) is a polymer compound, it is preferably a conjugated polymer from the standpoint of injection and transportation of charges. Here, the conjugated polymer is as defined above.

When the compound having a residue of a compound represented by the above-described formula (2) is a polymer compound, it prefreably contains at least one repeating unit represented by any of the above-described formulae (A), (B) and (C).

The polymer compound having as a repeating unit a residue which is an atomic group obtained by removing two R's from a group represented by -C(R')= in the above-described formula (2) includes polymer compounds having a repeating unit represenetd by the following formula (4): 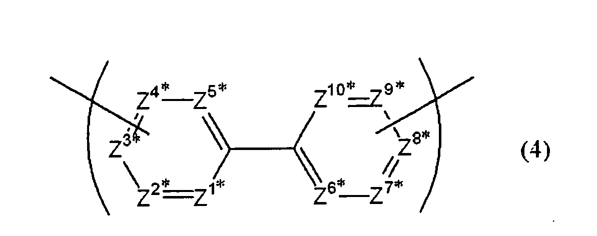 wherein one member of Z^{1*}, Z^{2*} and Z^{3*} represents -N= and two remainders thereof each represent -C(R")=; Z^{4*} and Z^{5*} each represent -C(R")=; one member of Z^{6*}, Z^{7*} and Z^{8*} represents -N= and two remainders thereof each represent -C(R")=; Z^{9*} and Z^{10*} represent -C(R")=; R" represents a hydrogen atom, an alkyl group, an alkoxy group, an aryl group, an aryloxy group, an arylalkyl group, an arylalkoxy group, an arylalkenyl group, an arylalkynyl group, an amino group, a substituted amino group, a halogen atom, an acyl group, an acyloxy group, a mono-valent heterocyclic group, a carboxyl group, a nitro group, or a cyano group, and one R" contained in Z^{1*}, Z^{2*}, Z^{3*}, Z^{4*} and Z^{5*} represents a connecting bond, and one R" contained in Z^{6*}, Z^{7*}, Z^{8*}, Z^{9*} and Z^{10*} represents a connecting bond; one or some or all of the hydrogen atoms contained in the group represented by R" may be substituted by a fluorine atom. Eight -C(R")= groups may be the same or different; when Z^{2*} and Z^{3*} each represent -C(R")=, two R"s contained in Z^{2*} and Z^{3*} may be combined together to form a benzene ring, and when Z^{3*} represents -C(R")=, two R"s contained in Z^{3*} and Z^{4*} may be combined together to form a benzene ring and two R"s contained in Z^{4*} and Z^{5*} may be combined together to form a benzene ring, providing that two or more combinations of Z^{2*} and Z^{3*}, Z^{3*} and Z^{4*}, and Z^{4*} and Z^{5*} do not simultaneously form a benzene ring; when Z^{7*} and Z^{8*} each represent -C(R")=, two R"s contained in Z^{7*} and Z^{8*} may be combined together to form a benzene ring, and when Z^{8*} represents -C(R")=, two R"s contained in Z^{8*} and Z^{9*} may be combined together to form a benzene ring and two R''s contained in Z^{9*} and Z^{10*} may be combined together to form a benzene ring, providing that two or more combinations of Z^{7*} and Z^{8*}, Z^{8*} and Z^{9*}, and Z^{9*} and Z^{10*} do not simultaneously form a benzene ring; the benzene ring formed by mutual combination of two R"s optionally has a substituent. or a repeating unit represented by the following formula (5) : 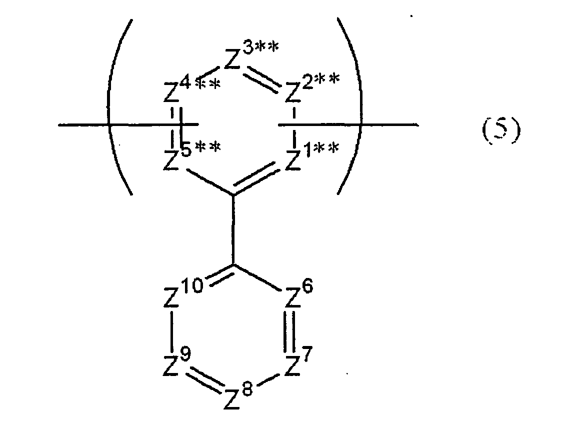 wherein one member of Z1^{**}, Z^{2**} and Z^{3**} represents -N= and two remainders thereof each represent -C(R''')=; Z^{4**} and Z^{5**} each represent -C(R''')=; R''' represents a hydrogen atom, an alkyl group, an alkoxy group, an aryl group, an aryloxy group, an arylalkyl group, an arylalkoxy group, an arylalkenyl group, an arylalkynyl group, an amino group, a substituted amino group, a halogen atom, an acyl group, an acyloxy group, a mono-valent heterocyclic group, a carboxyl group, a nitro group, or a cyano group, and two R' ' ' s contained in Z^{1**}, Z^{2**}, Z^{3**}, Z^{4**} and Z^{5**} each represent a connecting bond; Z⁶, Z⁷, Z⁸, Z⁹ and Z¹⁰ each have the same meaning as described above; one or some or all of the hydrogen atoms contained in the group represented by R' and R''' may be substituted by a fluorine atom; four -C(R')= groups may be the same or different; four -C(R''')= groups may be the same or different; when Z^{2**} and Z^{3**} each represent -C(R''')=, two R'''s contained in Z^{2**} and Z^{3**} may be combined together to form a benzene ring, and when Z^{3**} represents -C(R''')=, two R'''s contained in Z^{3**} and Z^{4**} may be combined together to form a benzene ring and two R'''s contained in Z^{4**} and Z^{5**} may be combined together to form a benzene ring, providing that two or more combinations of Z^{2**} and Z^{3**}, Z^{3**} and Z^{4**}, and Z^{4**} and Z^{5**} do not simultaneously form a benzene ring; when Z⁷ and Z⁸ each represent -C(R')=, two R's contained in Z⁷ and Z⁸ may be combined together to form a benzene ring, and when Z⁸ represents -C(R')=, two R's contained in Z⁸ and Z⁹ may be combined together to form a benzene ring and two R's contained in Z⁹ and Z¹⁰ may be combined together to form a benzene ring, providing that two or more combinations of Z⁷ and Z⁸, Z⁸ and Z⁹, and Z⁹ and Z¹⁰ do not simultaneously form a benzene ring; the benzene ring formed by mutual combination of two R's optionally has a substituent, and the benzene ring formed by mutual combination of two R'''s optionally has a substituent.

Regarding the -C(R'')= groups represented by eight members among Z^{1*}, Z^{2*}, Z^{3*}, Z^{4*}, Z^{5*}, Z^{6*}, Z^{7*}, Z^{8*}, Z^{9*} and Z^{10*} in the above-described formula (4), one moiety thereof represents a connecting bond, and the alkyl group, alkoxy group, aryl group, aryloxy group, arylalkyl group, arylalkoxy group, arylalkenyl group, arylalkynyl group, amino group, substituted amino group, halogen atom, acyl group, acyloxy group, mono-valent heterocyclic group, carboxyl group, nitro group and cyano group represented by the remaining R" groups have the same meaning as described above, and preferable are a hydrogen atom, alkyl group, alkoxy group, aryl group, aryloxy group, arylalkyl group, arylalkoxy group, halogen atom and mono-valent heterocyclic group, more preferable are a hydrogen atom, alkyl group, aryl group, arylalkyl group, halogen atom and mono-valent heterocyclic group, further preferable are a hydrogen atom, alkyl group and aryl group, particularly preferable are a hydrogen atom and alkyl group.

It is preferable that the position of -N= represented by one member among Z^{1*}, Z^{2*} and Z^{3*} and the position of - N= represented by one member among Z^{6*}, Z^{7*} and Z^{8*} are symmetrical in the above-described formula (4). Specifically, a case in which Z^{1*} and Z^{6*} represent -N= and Z^{2*}, Z^{3*}, Z^{7*} and Z^{8*} represent -C(R'')=, a case in which Z^{2*} and Z^{7*} represent -N= and Z^{1*}, Z^{3*}, Z^{6*} and Z^{8*} represent -C(R")=, and a case in which Z^{3*} and Z^{8*} represent -N= and Z^{1*}, Z^{2*}, Z^{6*} and Z^{7*} represent -C(R")= are preferable , and a case in which Z^{1*} and Z^{6*} represent -N= and Z^{2*}, Z^{3*}, Z^{7*} and Z^{8*} represent -C(R")= is more preferable.

In the polymer compound having a repeating unit represented by the above-described formula (4), it is preferable that two connecting bonds are obtained by removing R"s contained in Z^{3*} and Z^{8*}.

The polymer compound having a repeating unit represented by the above-described formula (4) includes polymer compounds having one or more members among repeating units represented by the following formulae.

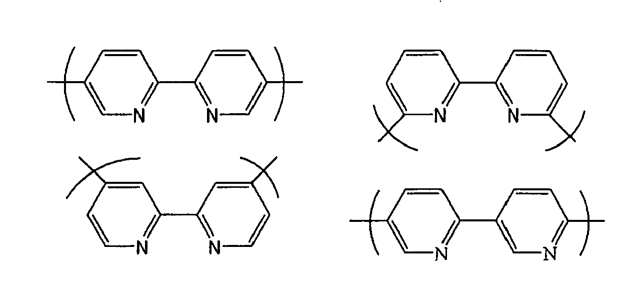

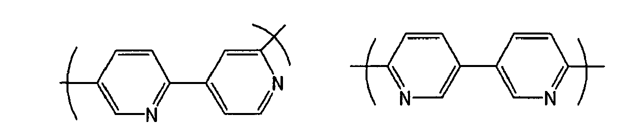

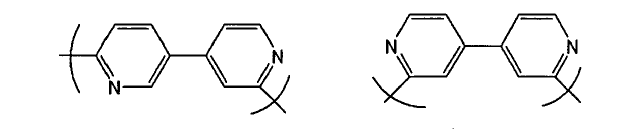

Regarding the -C(R''')= groups represented by four members among Z^{1**}, Z^{2**}, Z^{3**}, Z^{4**} and Z^{5**} in the above-described formula (5), two moieties represent a connecting bond, and the alkyl group, alkoxy group, aryl group, aryloxy group, arylalkyl group, arylalkoxy group, arylalkenyl group, arylalkynyl group, amino group, substituted amino group, halogen atom, acyl group, acyloxy group, mono-valent heterocyclic group, carboxyl group, nitro group or cyano group represented by the remaining R'''s have the same meaning as described above, and preferable are a hydrogen atom, alkyl group, alkoxy group, aryl group, aryloxy group, arylalkyl group, arylalkoxy group, halogen atom and mono-valent heterocyclic group, more preferable are a hydrogen atom, alkyl group, aryl group, arylalkyl group, halogen atom and mono-valent heterocyclic group, further preferable are a hydrogen atom, alkyl group and aryl group, particularly preferable are a hydrogen atom and alkyl group.

It is preferable that the position of -N= represented by Z^{1**}, Z^{2**} and Z^{3**} and the position of -N= represented by Z⁶, Z⁷ and Z⁸ are symmetrical in the above-described formula (5). Specifically, a case in which Z^{1**} and Z⁶ represent -N= and Z^{2**}, Z^{3**}, Z⁷ and Z⁸ represent - C(R''')=, a case in which Z^{2**} and Z⁷ represent -N= and Z^{1**}, Z^{3**}, Z⁶ and Z⁸ represent -C(R''')=, and a case in which Z^{3**} and Z⁸ represent -N= and Z^{1**}, Z^{2**}, Z⁶ and Z⁷ represent -C(R''')= are preferable, and a case in which Z^{1**} and Z⁶ represent -N= and Z^{2**}, Z^{3**}, Z⁷ and Z⁸ represent -C(R''')= is more preferable.

In the polymer compound having a repeating unit represented by the above-described formula (5), it is preferable that two connecting bonds are obtained by removing R'''s contained in Z^{2**} and Z^{4**}, or Z^{2**} and Z^{4**}.

The polymer compound having a repeating unit represented by the above-described formula (5) includes polymer compounds having repeating units represented by the following formulae.

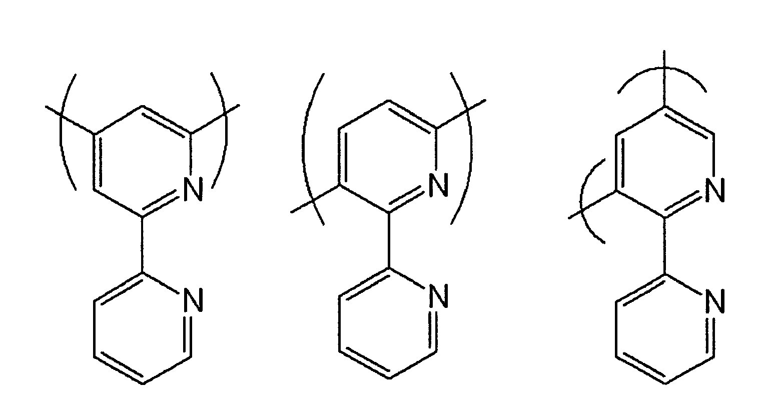

The compounds represented by the above-described formula (2) and the compounds having residues of compounds represented by the above-described formula (2) may be used singly or in combination of two or more.

### <Preferable Embodiments>

The composition of the present invention preferably includes a composition comprising a compound represented by the above-described formula (1) and a compound represented by the above-described formula (2); a composition comprising a compound having a residue of a compound represented by the above-described formula (1) and a compound represented by the above-described formula (2) or a compound having a residue of a compound represented by the above-described formula (2) wherein the compound having a residue of a compound represented by the above-described formula (1) is a polymer compound having a repeating unit containing a residue of a compound represented by the above-described formula (1); a composition comprising a compound represented by the above-described formula (1) or a compound having a residue of a compound represented by the above-described formula (1) and a compound having a residue of a compound represented by the above-described formula (2) wherein the compound having a residue of a compound represented by the above-described formula (2) is a polymer compound having a repeating unit containing a residue of a compound represented by the above-described formula (2); and a composition comprising a compound having a residue of a compound represented by the above-described formula (1) and a compound having a residue of a compound represented by the above-described formula (2) wherein the compound having a residue of a compound represented by the above-described formula (1) is a polymer compound having a repeating unit containing a residue of a compound represented by the above-described formula (1) and the compound having a residue of a compound represented by the above-described formula (2) is a polymer compound having a repeating unit containing a residue of a compound represented by the above-described formula (2), from the standpoint of the luminance half life of an organic electroluminescent device.

In the composition of the present invention, the total weight of the compound represented by the above-described formula (2) and the residue of a compound represented by the above-described formula (2) is preferably 0.1 to 1000 parts by weight, more preferably 0.5 to 1000 parts by weight with respect to 100 parts by weight of the total weight of the compound represented by the above-described formula (1) and the residue of a compound represented by the above-described formula (1), from the standpoint of the luminance half life of an organic electroluminescent device.

In the composition of the present invention, the sum of the compound represented by the above-described formula (2) and the compound having a residue of a compound represented by the above-described formula (2) is preferably 0.5 to 100 parts by weight, more preferably 0.5 to 50 parts by weight with respect to 100 parts by weight of the sum of the compound represented by the above-described formula (1) and the compound having a residue of a compound represented by the above-described formula (1), from the standpoint of compatibility.

The composition of the present invention may contain components (other components) other than the compound represented by the above-described compound (1), the compound having a residue of a compound represented by the above-described compound (1), the compound represented by the above-described compound (2) and the compound having a residue of a compound represented by the above-described compound (2), and for example, at least one selected from the group consisting of light emitting materials, hole transporting materials and electron transporting materials may be contained. The other components may each be used singly or in combination of two or more.

The above-described light emitting material includes low molecular weight fluorescence emitting materials, phosphorescence emitting materials and the like, and examples thereof include naphthalene derivatives, anthracene and derivatives thereof, perylene and derivatives thereof, dyes such as polymethine dyes, xanthene dyes, coumarine dyes, cyanine dyes and the like; metal complexes having 8-hydroxyquinoline as a ligand; metal complexes having a 8-hydroxyquinoline derivative as a ligand; other fluorescent metal complexes, aromatic amines, tetraphenylcyclopentadiene and derivatives thereof, tetraphenylbutadiene and derivatives thereof, and fluorescent materials of low molecular weight compounds such as stilbene, silicon-containing aromatic, oxazole, furoxane, thiazole, tetraarylmethane, thiadiazole, pyrazole, metacyclophane, acetylene and the like; metal complexes such as an iridium complex, platinum complex and the like; triplet emitting complexes, and the like. Additionaly, those described in JP-A No. 57-51781, JP-A No. 59-194393 and the like are also mentioned.

In the composition of the present invention, the proportion of the light emitting material is preferably 1 to 500 parts by weight, more preferably 3 to 400 parts by weight, particularly preferably 3 to 300 parts by weight with respect to 100 parts by weight of the total weight of the compound represented by the above-described formula (1) and the residue of a compound represented by the above-described formula (1), from the standpoint of the chromaticity of an organic electroluminescent device.

The above-described hole transporting material includes polyvinylcarbazole and its derivatives, polysilane and its derivatives, polysiloxane derivatives having an aromatic amine in a side chain or main chain, pyrazoline derivatives, arylamine derivatives, stilbene derivatives, triphenyldiamine derivatives, polyaniline and its derivatives, polythiophene and its derivatives, polypyrrole and its derivatives, poly(p-phenylenevinylene) and its derivatives, poly(2,5-thienylenevinylene) and its derivatives, and the like. Additionally, those described in JP-A Nos. 63-70257 and 63-175860, JP-A Nos. 2-135359, 2-135361, 2-209988, 3-37992 and 3-152184 are also mentioned.

In the composition of the present invention, the proportion of the hole transporting material is preferably 3 to 500 parts by weight, more preferably 3 to 400 parts by weight, particularly preferably 3 to 300 parts by weight with respect to 100 parts by weight of the total weight of the compound represented by the above-described formula (1) and the residue of a compound represented by the above-described formula (1), from the standpoint of charge balance.

The above-described electron transporting material includes oxadiazole derivatives, anthraquinodimethane and its derivatives, benzoquinone and its derivatives, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, metal complexes of 8-hydroxyquinoline and its derivatives, polyquinoline and its derivatives, polyquinoxaline and its derivatives, polyfluorene and its derivatives, and the like. Additionally, those described in JP-A Nos. 63-70257 and 63-175860, JP-A Nos. 2-135359, 2-135361, 2-209988, 3-37992 and 3-152184 are mentioned.

In the composition of the present invention, the proportion of the electron transporting material is preferably 3 to 500 parts by weight, more preferably 3 to 400 parts by weight, particularly preferably 3 to 300 parts by weight with respect to 100 parts by weight of the total weight of the compound represented by the above-described formula (1) and the residue of a compound represented by the above-described formula (1), from the standpoint of charge balance.

The composition of the present invention can be made into a solution or dispersion (hereinafter, referred to simply as "solution") by inclusion of an organic solvent. By this, film formation can be carried out by an application method. This solution is called, in general, an ink, liquid composition or the like.

The above-described organic solvent includes chlorine-based solvents such as chloroform, methylene chloride, 1,2-dichloroethane, 1,1,2-trichloroethane, chlorobenzene, o-dichlorobenzene and the like, ether solvents such as tetrahydrofuran, dioxane and the like, aromatic hydrocarbon solvents such as toluene, xylene, trimethylbenzene, mesitylene and the like, aliphatic hydrocarbon solvents such as cyclohexane, methylcyclohexane, n-pentane, n-hexane, n-heptane, n-octane, n-nonane, n-decane and the like, ketone solvents such as acetone, methyl ethyl ketone, cyclohexanone and the like, ester solvents such as ethyl acetate, butyl acetate, methyl benzoate, ethylcellosolve acetate and the like, polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, 1,2-hexane diol and the like and derivatives thereof, alcohol solvents such as methanol, ethanol, propanol, isopropanol, cyclohexanol and the like, sulfoxide solvents such as dimethyl sulfoxide and the like, amide solvents such as N-methyl-2-pyrrolidone, N,N-dimethylformamide, and the like. The above-described solvents may be used singly or in combination of two or more. Among the above-described solvents, organic solvents having a structure containing a benzene ring and having a melting point of 0°C or lower and a boiling point of 100°C or higher are preferably contained from the standpoint of viscosity, film formability and the like.

In lamination and film formation from the composition of the present invention when the composition of the present invention contains the above-described organic solvent, it may be advantageous to only remove the organic solvent by drying after application of the composition of the present invention, and this is very advantageous for production. In drying, drying may be effected under heating at about 50 to 150°C, alternatively, drying may be carried out under reduced pressure of about 10⁻³ Pa.

For the above-described lamination and film formation, application methods such as a spin coat method, casting method, micro gravure coat method, gravure coat method, bar coat method, roll coat method, wire bar coat method, dip coat method, slit coat method, capillary coat method, spray coat method, screen printing method, flexo printing method, offset printing method, inkjet print method, nozzle coat method and the like can be used.

When the composition of the present invention contains the above-described organic solvent, the preferable viscosity of the above-described solution is preferably in the range of 0.5 to 500 mPa·s at 25°C though it varies depeding on the printing method, and when a liquid composition passes through a discharge apparatus such as in an inkjet print method and the like, the viscosity at 25°C is preferably in the range of 0.5 to 20 mPa·s, for preventing clogging and flying curving in discharging.

### <Organic electroluminescent device>

The organic electroluminescent device of the present invention is obtained by using the above-described composition, and usually, has an anode, a cathode, and a layer obtained by using the composition of the present invention disposed between the anode and the carhode, and it is prefeable that the layer obtained by using the composition is a light emitting layer. A case in which the layer obtained by using the composition of the present invention is a light emitting layer will be illustrated as one example, below.

The constitution of the organic electroluminescent device of the present invention includes the following structures a) to d).
a) anode/light emitting layer/cathode
b) anode/hole transporting layer/light emitting layer/cathode
c) anode/light emitting layer/electron transporting layer/cathode
d) anode/hole transporting layer/light emitting layer/electron transporting layer/cathode
(Here, "/" means adjacent lamination of layers, the same shall apply hereinafter)

The light emitting layer is a layer having a function of emitting light, the hole transporting layer is a layer having a function of transporting holes, and the electron transporting layer is a layer having a function of transporting electrons. The hole transporting layer and electron transporting layer are collectively called a charge transporting layer. The hole transporting layer adjacent to the light emitting layer is called an interlayer layer in some cases.

Lamination and fiml formation of each layer can be performed from a solution. For lamination and film formation from a solution, application methods such as a spin coat method, casting method, micro gravure coat method, gravure coat method, bar coat method, roll coat method, wire bar coat method, dip coat method, slit coat method, capillary coat method, spray coat method, screen printing method, flexo printing method, offset printing method, inkjet print method, nozzle coat method and the like can be used.

The thickness of a light emitting layer may be advantageously regulated so as to give appropriate values of driving voltage and light emission efficiency, and is usually 1 nm to 1 µm, preferably 2 nm to 500 nm, further preferably 5 nm to 200 nm.

When the organic electroluminescent device of the present invention has a hole transporting layer, the hole transporting material to be used is as described above. Formation of a hole transporting layer may be carried out by any methods, and when the hole transporting material is a low molecular weight compound, film formation from a mixed solution with a polymer bindes is preferable. When the hole transporting material is a polymer compound, film formation from a solution is preferable. For film formation from a solution, the methods exemplified as the above-described application method can be used.

As the polymer binder to be mixed, those not extremely disturbing charge transportation and showing no strong absorption for visible light are preferable. The polymer binder includes polycarbonates, polyacrylates, polymethyl acrylate, polymethyl methacrylate, polystyrene, polyvinyl chloride, polysiloxane and the like.

The thickness of the hole transporting layer may be advantageously selected so as to give suitable vaslues of driving voltage and light emission efficiency, and a thickness at least causing no formation of pin holes is necessary, and when the thickness is too large, the driving voltage of a device increases undesirably. Therefore, the thickness of the hole transporting layer is usually 1 nm to 1 µm, preferably 2 nm to 500 nm, and further preferably 5 nm to 200 nm.

When the organic electroluminescene device of the present invention has an electron transporting layer, the electron transporting material to be used is as described above. Formation of the electron transporting layer may be carried out by any methods, and when the electron transporting material is a low molecular weight compound, a vacuum vapor deposition method from a powder and a method of film formation from a solution or melted condition are preferable. When the electron transporting material is a polymer compound, a method of film formation from a solution or melted condition is preferable. For film formation from a solution or melted condition, a polymer binder may be used together. For film formation from a solution, the methods exemplified as the above-described application method can be used.

As the polymer binder to be mixed, those not extremely disturbing charge transportation and showing no strong absorption for visible light are preferable. The polymer binder includes poly(N-vinylcarbazole), polyaniline and derivatives thereof, polythiophene and derivatives thereof, poly(p-phenylenevinylene) and derivatives thereof, poly(2,5-thienylenevinylene) and derivatives thereof, polycarbonate, polyacrylate, polymethyl acrylate, polymethyl methacrylate, polystyrene, polyvinyl chloride, polysiloxane and the like.

The thickness of the electron transporting layer may be advantageously selected so as to give suitable values of driving voltage and light emission efficiency, and a thickness at least causing no formation of pin holes is necessary, and when the thickness is too large, the driving voltage of a device increases undesirably. Therefore, the thickness of the electron transporting layer is usually 1 nm to 1 µm, preferably 2 nm to 500 nm, further preferably 5 nm to 200 nm.

Among charge transporting layers disposed adjacent to an electrode, those having a function of improving charge injection efficiency from an electrode and having an effect of lowering the driving voltage of a device are, in particularly, called charge injection layers (hole injection layer, electron injection layer) in some cases. Further, for improving close adherence with an electrode and improving charge injection from an electron, the above-mentioned charge injection layer or insulation layer may be disposed adjacent to the electrode, alternatively, for improving close adherence of an interface and preventing mixing, a thin buffer layer may be inserted into an interface of a charge transporting layer and a light emitting layer. The order and number of layers to be laminated, and the thickness of each layer may be appropriately determined in view of light emission efficiency and device life.

The organic electroluminescent device having a charge injection layer includes those having the following structures e) to p).
e) anode/charge injection layer/light emitting layer/cathode
f) anode/light emitting layer/charge injection layer/cathode
g) anode/charge injection layer/light emitting layer/charge injection layer/cathode
h) anode/charge injection layer/hole transporting layer/light emitting layer/cathode
i) anode/hole transporting layer/light emitting layer/charge injection layer/cathode
j) anode/charge injection layer/hole transporting layer/light emitting layer/charge injection layer/cathode
k) anode/charge injection layer/light emitting layer/charge transporting layer/cathode
1) anode/light emitting layer/electron transporting layer/charge injection layer/cathode
m) anode/charge injection layer/light emitting layer/electron transporting layer/charge injection layer/cathode
n) anode/charge injection layer/hole transporting layer/light emitting layer/charge transporting layer/cathode
o) anode/hole transporting layer/light emitting layer/electron transporting layer/charge injection layer/cathode
p) anode/charge injection layer/hole transporting layer/light emitting layer/electron transporting layer/charge injection layer/cathode

The charge injection layer includes a layer containing an electric conductive polymer, a layer disposed between an anode and a hole transporting layer and containing a material having ionization potential of a value between an anode material and a hole transporting material contained in the hole transporting layer, a layer disposed between a cathode and an electron transporting layer and containing a material having electron affinity of a value between a cathode material and an electron transporting material contained in the electron transporting layer, and the like.

When the above-mentioned charge injection layer is a layer containing an electric conductive polymer, the electric conductivity of the electric conductive polymer is preferably 10⁻⁵ S/cm to 10³ S/cm, and for decreasing leak current between light emission picture elements, it is more preferably 10⁻⁵ S/cm to 10² S/cm, particularly preferably 10⁻⁵ S/cm to 10¹ S/cm. For satisfying such a range, the electric conductive polymer may be doped with a suitable amount of ions.

As the kind of ions to be doped, an anion is used in the case of a hole injection layer and a cation is used in the case of an electron injection layer. The anion includes a polystyrenesulfonic ion, alkylbenzenesulfonic ion, camphorsulfonic ion and the like, and the cation includes a lithium ion, sodium ion, potassium ion, tetrabutylammonium ion and the like.

The thickness of the charge injection layer is, for example, 1 nm to 100 nm, preferably 2 nm to 50 nm.

The material to be used in the charge injection layer may be appropriately selected depending on a relation with materials of an electrode and an adjacent layer, and mentioned are electric conductive polymers such as polyaniline and its derivatives, polythiophene and its derivatives, polypyrrole and its derivatives, polyphenylenevinylene and its derivatives, polythienylenevinylene and its derivatives, polyquinoline and its derivatives, polyquinoxaline and its derivatives, polymers containing an aromatic amine structure on the main chain or side chain, and the like, and metal phthalocyanines (copper phthalocyanine and the like), carbon and the like.

The insulation layer has a function of making charge injection easy. The average thickness of this insulation layer is usually 0.1 to 20 nm, preferably 0.5 to 10 nm, more preferably 1 to 5 nm.

As the material to be used in the insulation layer, metal fluorides, metal oxides, organic insulating materials and the like are mentioned.

The organic electroluminescent device having an insulation layer includes thoese having the followiing structures q) to ab).
q) anode/insulation layer/light emitting layer/cathode
r) anode/light emitting layer/insulation layer/cathode
s) anode/insulation layer/light emitting layer/insulation layer/cathode
t) anode/insulation layer/hole transporting layer/light emitting layer/cathode
u) anode/hole transporting layer/light emitting layer/insulation layer/cathode
v) anode/insulation layer/hole transporting layer/light emitting layer/insulation layer/cathode
w) anode/insulation layer/light emitting layer/electron transporting layer/cathode
x) anode/light emitting layer/electron transporting layer/insulation layer/cathode
y) anode/insulation layer/light emitting layer/electron transporting layer/insulation layer/cathode
z) anode/insulation layer/hole transporting layer/light emitting layer/electron transporting layer/cathode
aa) anode/hole transporting layer/light emitting layer/electron transporting layer/insulation layer/cathode
ab) anode/insulation layer/hole transporting layer/light emitting layer/electron transporting layer/insulation layer/cathode

The substrate for forming an organic electroluminescent device of the present invention may advantageously be one which does not change in forming an electrode and an organic layer, and for example, substrates of made of glass, plastic, polymer film, silicon and the like are mentioned. In the case of an opaque substrate, it is preferable that an electrode nearer to the substrate and the opposite electrode are transparent or semi-transparent.

In the present invention, it is usually preferable that at least one of electrodes consisting of an anode and cathode is transparent or semi-transparent, and the anode side is transparent or semi-transparent.

As the material of the anode, an electric conductive metal oxide film, semi-transparent metal film and the like are used, and specifically, films (NESA and the like) formed using electric conductive inorganic compounds composed of indium oxide, zinc oxide, tin oxide, and composite thereof: indium·tin·oxide (ITO), indium·zinc·oxide and the like, and gold, platinum, silver, copper and the like are used. As the anode, organic transparent electric conductive films made of polyaniline and its derivatives, polythiophene and its derivatives, and the like may be used. For making electric charge injection easy, a layer made of a phthalocyanine derivative, electric conductive polymer, carbon and the like, or a layer made of a metal oxide, metal fluoride, organic insulation material and the like, may be provided on an anode.

As the method for fabricating an anode, a vacuum vapor deposition method, sputtering method, ion plating method, plating method and the like are mentioned.

The thickness of an anode can be appropriately selected in view of light transmission and electric conductivity, and it is usually 10 nm to 10 µm, preferably 20 nm to 1 µm, further preferably 50 nm to 500 nm.

As the material of a cathode, materials of small work function are preferable, and use is made of metals such as lithium, sodium, potassium, rubidium, cesium, beryllium, magnesium, calcium, strontium, barium, aluminum, scandium, vanadium, zinc, yttrium, indium, cerium, samarium, europium, terbium, ytterbium and the like, alloys composed of two or more of them, or alloys composed of at least one of them and at least one of gold, silver, platinum, copper, manganese, titanium, cobalt, nickel, tungsten and tin, and graphite or graphite intercalation compounds and the like.

As the method for fabricating a cathode, a vacuum vapor deposition method, sputtering method, a laminate method of thermally press bonding a metal film, and the like are used.

The thickness of a cathode can be appropriately selected in view of electric conductivity and durability, and it is usually 10 nm to 10 µm, preferably 20 nm to 1 µm, further preferably 50 nm to 500 nm.

A layer made of an electric conductive polymer, or a layer made of a metal oxide, metal fluoride, organic insulation material and the like, may be provided between a cathode and a light emitting layer, or between a cathode and an electron transporting layer, and after fabrication of a cathode, a protective layer for protecting the organic electroluminscence device may be installed. For use of the organic electroluminscence device stably for a long period of time, it is preferable to install a protective layer and/or protective cover, for protecting a device from outside.

As the protective layer, resins, metal oxides, metal fluorides, metal borides and the like can be used. As the protective cover, a glass plate, and a plastic plate having a surface which has been subjected to a low water permeation treatment, and the like can be used, and a method in which the cover is pasted to a device substrate with a thermosetting resin or photo-curing resin to attain sealing is suitably used. When a space is kept using a spacer, blemishing of a device can be prevented easily. If an inert gas such as nitrogen, argon and the like is filled in this space, oxidation of a cathode can be prevented, further, by placing a drying agent such as barium oxide and the like in this space, it becomes easy to suppress moisture adsorbed in a production process from imparting damage to the device.

The luminance half life of an organic electroluminescent device can be improved by applying a composition of the present invention, to form a film, then, drying this under suitable conditions. For drying, the film is preferably heated at 50 to 200°C, more preferably heated at 50 to 150°C, further preferably heated at 50 to 130°C, particularly preferably heated at 50 to 110°C. Heating at 70 to 110°C is especially prpeferable. The time of the above-described heating is preferably 1 minte to 24 hours, more preferably 3 minutes to 6 hours, particularly preferably 5 minutes to 3 hours. The amosphere in the above-described heating is preferably under an inert gas or under a reduced pressure of about 10⁻³Pa. Here, the inert gas includes gases of elements belongind to Group XVIII element, and a nigrogen gas.

The composition and the organic electroluminescent device of the present invention are useful for planar light sources such as curved light sources, flat light sources and the like (for example, illumination and the like); displays such as segment displays (for example, segment type display and the like), dot matrix displays (for example, dot matrix flat display and the like), liquid crystal displays (for example, liquid crystal display, backlight of liquid crystal display, and the like); etc. The composition of the present invention is suitable as a material used for fabrication of these apparatuses, and additionally, also useful as a dye for laser, a material for organic solar batteries, an organic semiconductor for organic transistors, a material for conductive films such as electric conductive films, organic semiconductor films and the like, a material for luminescent films emitting fluorescence, a material for polymer electric field effect transistors, and the like.

For obtaining light emission in the form of plane using an organic elextroluminescence device of the present invention, it may be advantages to place a planar anode and a planar cathode so as to overlap. For obtaining light emission in the form of pattern, there are a method in which a mask having a window in the form of pattern is placed on the surface of the above-mentioned planar light emitting device, and a method in which either an anode or a cathode, or both electrodes are formed in the form pattern.

By forming a pattern by any of these methods, and placing several electrodes so that ON/OFF is independently possible, a display device of segment type is obtained which can display digits, letters, simple marks and the like. Further, for providing a dot matrix device, it may be permissible that both an anode and a cathode are formed in the form of stripe, and placed so as to cross. By a method in which several polymer compounds showing different emission colors are painted separately or a method in which a color filter or a fluorescence conversion filter is used, partial color display and multi-color display are made possible. In the case of a dot matrix device, passive driving is possible, and active driving may also be carried out in combination with TFT and the like. These display devices can be used as a display of a computer, television, portable terminal, cellular telephone, car navigation, video camera view finder, and the like.

### EXAMPLES

The present invention will be illustrated specifically using examples.

### <Synthesis Example 1> (Synthesis of low molecular weight compound A)

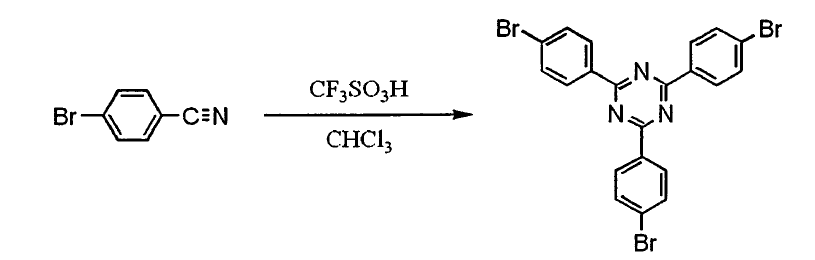

Under a nitrogen atmosphere, 100 g (0.65 mol) of trifluoromethanesulfonic acid was charged, and stirred at room temperature. To the resultant reaction liquid was added dropwise a solution prepared by dissolving 61.93 g (0.33 mol) of 4-bromobenzonitrile in 851 ml of dehydrated chloroform. The resultant solution was heated up to 95°C, and stirred while heating, then, cooled down to room temperature, and to this was added a dilute ammonia aqueous solution under an ice bath. The resultant solid was filtrated, washed with water, then, washed with diethyl ether, and dried under reduced pressure to obtain 47.8 g of a white crystal.

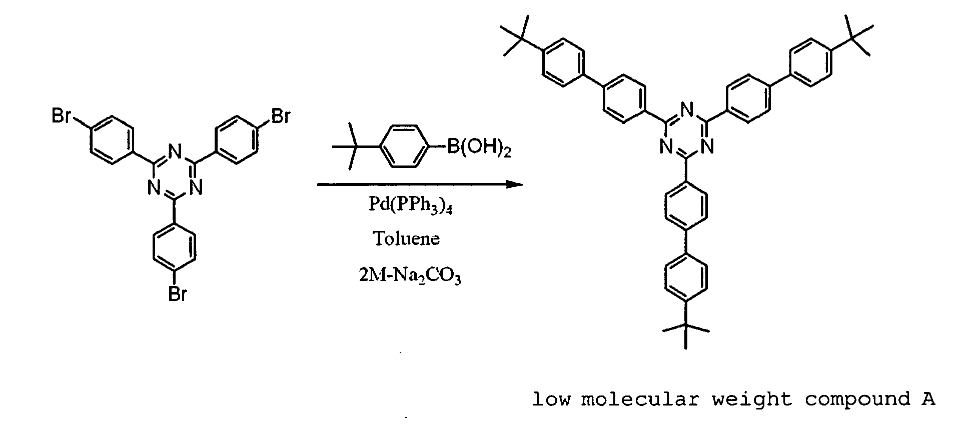

Under a nitrogen atmosphere, 8.06 g (14.65 mmol) of the resultant white crystal, 9.15 g (49.84 mmol) of 4-t-butylphenylboronic acid, 1.54 g (1.32 mmol) of Pd(PPh₃)₄, 500 ml of toluene previously bubbled with nitrogen and 47.3 ml of ethanol previously bubbled with nitrogen were charged, stirred, heated, and refluxed. Into the resultant reaction liquid, 47.3 ml of a 2M sodium carbonate aqueous solution previously bubbled with nitrogen was dropped, further heated, and refluxed. The resultant reaction liquid was allowed to cool, then, liquid-separated, and the aqueous phase was removed, and the organic phase was washed with dilute hydrochloric acid and water in this order, and liquid-separated. The organic phase was dried over anhydrous magnesium sulfate, filtrated, and concentrated. The resultant coarse product was passed through a silica gel column, and to the resultant filtrate was added acetonitrile. The resultant crystal was dried under reduced pressure to obtain 8.23 g of a white crystal (hereinafter, referred to as "low molecular weight compound A").
¹H-NMR (270 MHz/CDCl₃):
δ 1.39 (s, 27H), 7.52 (d, 6H), 7.65 (d, 6H), 7.79 (d, 6H), 8.82 (d, 6H)

### <Synthesis Example 2> (Synthesis of low molecular weight compound B)

Into a 300 ml four-necked flask was charged 8.08 g (20.0 mmol) of 1,4-dihexyl-2,5-dibromobenzene, 12.19 g (48.0 mmol) of bis(pinacolate)diboron and 11.78 g (120.0 mmol) of potassium acetate, and purging with argon was performed. Dehydrated 1,4-dioxane (100 ml) was charged, and deaeration with argon was performed. [1,1'-bis(diphenylphosphino)ferrocene]dichloropalladium(II) (0.98 g, 1.2 mmol) was charged, and deaeration with argon was further performed. Refluxing was carried out while heating for 6 hours, to obtain a dark brown slurry. Toluene and ion exchanged water were added, liquid-separation was performed, and washing with ion exchanged water was performed. Anhydrous sodium sulfate and activated carbon were added, and the mixture was filtrated through a funnel pre-coated with celite. The filtrate was concentrated to obtain 11.94 g of a dark brown crystal. Re-crystallization from n-hexane was carried out, and the crystal was washed with methanol. The resultant crystal was dried under reduced pressure, to obtain 4.23 g of 1,4-dihexylphenyl-2,5-boronic acid pinacol ester (hereinafter, referred to as "low molecular weight compound B") as a while needle crystal in a yield of 42.4%.
¹H-NMR (300 MHz/CDCl₃):
δ 0.95 (t, 6H), 1.39 to 1.42 (bd, 36H), 1.62 (m, 4H), 2.88 (t, 4H), 7.59 (bd, 2H)
LC/MS (ESI posi KCl addition): [M+K]⁺ 573

### <Synthesis Example 3> (Synthesis of low molecular weight compound C)

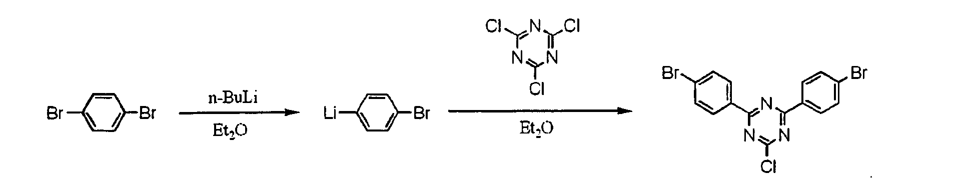

Under a nitrogen atmosphere, a solution containing 27.1 g (114.97 mmol) of 1,4-dibromobenzene in 217 ml of dehydrated diethyl ether was cooled down to -66°C. Into the resultant suspension, 37.2 ml (103.04 mmol) of a 2.77M n-butyllithium hexane solution was dropped over a period of 2 hours at -66°C or lower, then, the mixture was stirred at the same temperature for 1 hour, to prepare a lithium reagent.

Under a nitrogen atmosphere, a suspension containing 10.0 g (54.23 mmol) of cyanuric chloride in 68 ml of dehydrated diethyl ether was cooled down to -50°C, the above-described lithium reagent was added slowly over a period of 45 minutes at -35°C or lower, then, the mixture was heated up to room temperature, and reacted at room temperature. The resultant product was filtrated, and dried under reduced pressure. The resultant solid (16.5 g) was purified to obtain 13.2 g of a needle crystal.

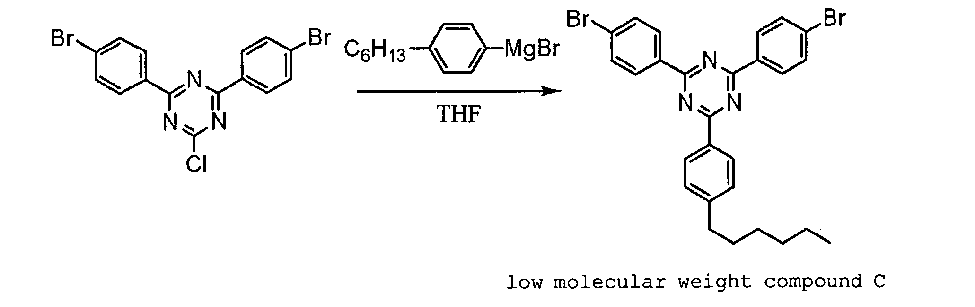

Under a nitrogen atmosphere, to a suspension prepared by adding 65 ml of dehydrated tetrahydrofuran to 1.37 g (56.4 mmol) of magnesium was gradually added a solution containing 14.2 g (59.2 mmol) of 4-hexylbromobenzene in 15 ml of dehydrated tetrahydrofuran, and the mixture was heated, and stirred under reflux. The resultant reaction liquid was allowed to cool, then, 0.39 g (16.3 mmol) of magnesium was additionally added, and the mixture was heated again, and reacted under reflux to prepare a Grignard reagent.

Under a nitrogen atmosphere, to a suspension containing 12.0 g (28.2 mmol) of the above-described needle crystal in 100 ml dehydrated tetrahydrofuran was added the above-described Grignard reagent, and the mixture was heated, and stirred under reflux. The resultant reaction liquid was allowed to cool, then, washed with a dilute hydrochloric acid aqueous solution, liquid-separation was performed, and the aqueous phase was extracted with diethyl ether. The resultant organic phases were combined, washed with water, then, liquid-separation was performed, the organic phase was dried over anhydrous magnesium sulfate, filtrated, and concentrated. The resultant white solid was purified in a silica gel column, and further recrystallized, to obtain 6.5 g of a white solid (hereinafter, referred to as "low molecular weight compound C").
¹H-NMR (400 MHz/CDCl₃):
δ 0.90 (t, 3H), 1.31 to 1.34 (m, 6H), 1.69 (m, 2H), 2.73 (t, 2H), 7.37 (d, 2H), 7.69 (d, 4H), 8.59 to 8.64 (m, 6H) LC/MS (APCI posi): [M+H]⁺ 566

### <Synthesis Example 4> (Light emitting material A: Synthesis of iridium complex)

A compound was synthesized according to a synthesis method described in WO 02/066552. That is, under a nitrogen atmosphere, 2-bromopyridine and 1.2 equivalent of 3-bromophenylboric acid were subjected to the Suzuki coupling (catalyst:
tetrakis(triphenylphosphine)palladium(0), base: 2M sodium carbonate aqueous solution, solvent: ethanol, toluene) to obtain 2-(3'-bromophenyl)pyridine represented by the following formula:

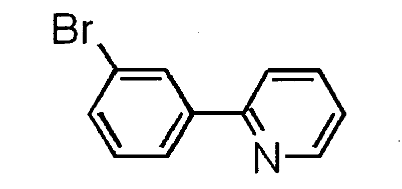

Next, under a nitrogen atmosphere, tribromobenzene and 2.2 equivalent of 4-tert-butylphenylboric acid were subjected to the Suzuki coupling (catalyst: tetrakis(triphenylphosphine)palladium(0), base: 2M sodium carbonate aqueous solution, solvent: ethanol, toluene) to obtain a bromo compound represented by the following formula:

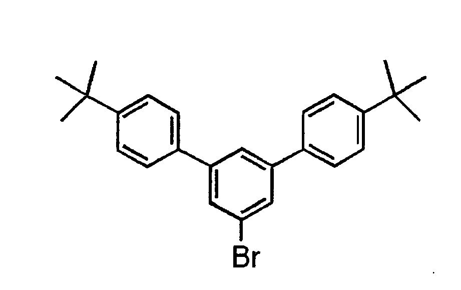

Under a nitrogen atmosphere, this bromo compound was dissolved in anhydrous THF, then, cooled down to -78°C, and small excess of tert-butyllithium was dropped. While cooling, B(OC₄H₉)₃ was further dropped, and reacted at room temperature. The resultant reaction liquid was post-treated with a 3M hydrochloric acid solution, to obtain a boric acid compound represented by the following formula: 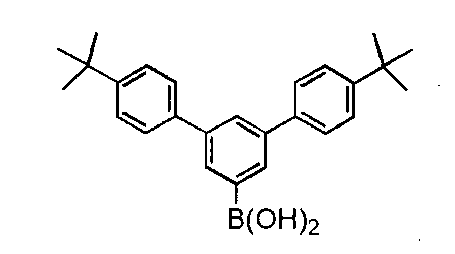 2-(3'-bromophenyl)pyridine and 1.2 equivalent of the above-described boric acid compound were subjected to the Suzuki coupling (catalyst:
tetrakis(triphenylphosphine)palladium(0), base: 2M sodium carbonate aqueous solution, solvent: ethanol, toluene) to obtain a ligand (namely, compound acting as ligand) represented by the following formula:

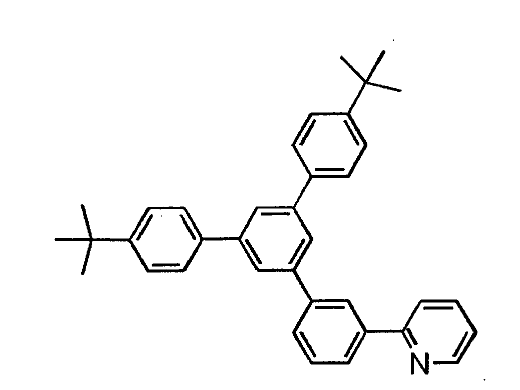

Under an argon atmosphere, the above-described ligand, 4 equivalent of IrCl₃·3H₂O, 2-EtOEtOH and ion exchanged water were charged, and refluxed. The deposited solid was filtrated under suction. The resultant solid was washed with ethanol and ion exchanged water in this order, then, dried to obtain a yellow powder represented by the following formula:

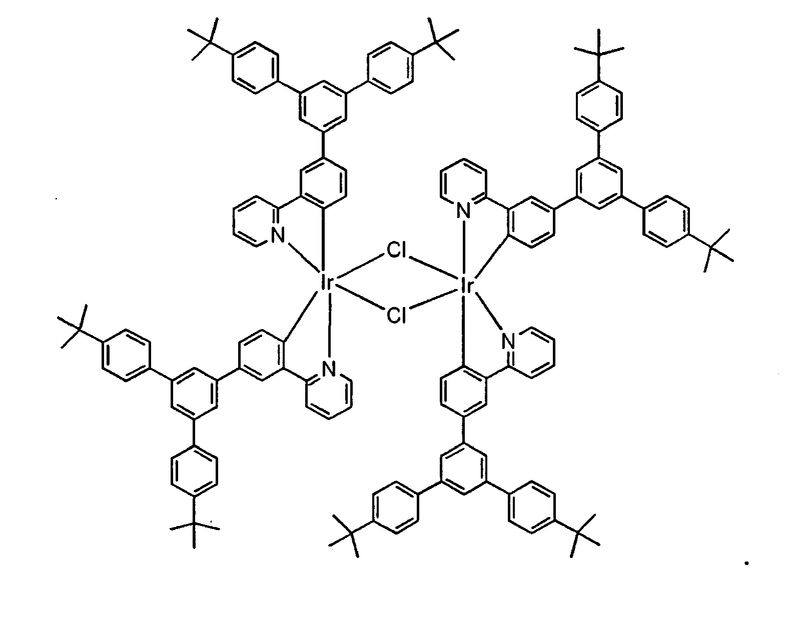

Under an argon atmosphere, to the above-described yellow powder was added 2 equivalent of the above-described ligand, and the mixture was heated in a glycol type solvent to obtain an iridium complex (hereinafter, referred to as "light emitting material A") represented by the following formula: 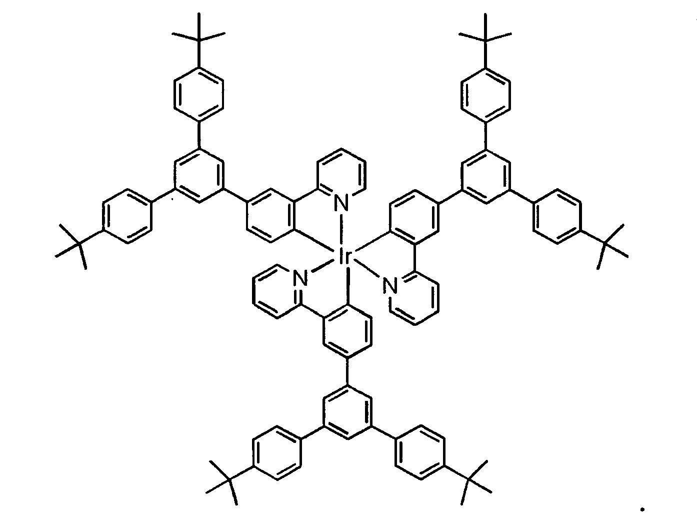 ¹H-NMR (300 MHz/CDCl₃):
δ 1..37 (s, 54H), 6.90 (t, 3H), 7.35 (d, 3H), 7.48 (d, 12H), 7.57 (d, 6H), 7.64 (d, 12H), 7.55 to 7.70 (m, 6H), 7.78 (s, 6H), 8.00 (d, 3H), 8.05 (s, 3H) LC/MS (APCI posi): [M+H]⁺ 1677

### <Synthesis Example 5> (Synthesis of low molecular weight compound D)

2-acetylpyridine (10.0 g, 82.6 mmol) and 2-methyl-3-dimethylaminopropenal (9.81 g, 86.7 mmol) were dissolved in tetrahydrofuran (126 ml), and potassium t-butoxide (9.37 g, 86.7 mmol) was added and the mixture was stirred at 60°C for 30 minutes. To the resultant mixed liquid was added ammonium acetate (67.68 g, 866.8 mmol) and acetic acid (83 ml), and the mixture was stirred at 60°C for 2 hours.
Then, the temperature in the system was heated up to 105°C, and the mixture was stirred for 3 hours while distilling tetrahydrofuran off. The resultant reaction liquid was allowed to cool, then, to this was added 200 ml of a 25 wt% sodium hydroxide aqueous solution, and the mixture was extracted with 300 ml of ethyl acetate four times in total. The resultant extraction liquids (organic layers) were combined, and dried over anhydrous sodium sulfate, and this was filtrated, then, concentrated. The resultant concentrated residue was purified by silica gel column chromatography (silica gel 300 g, eluate: hexane/ethyl acetate = 5/1 (volume ratio)), and concentrated to obtain 9.16 g (yield 65.2%) of a low molecular weight compound D represented by the following formula: 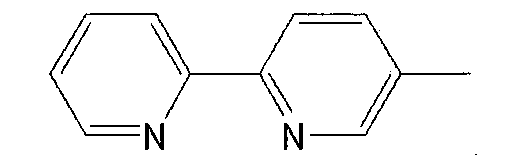 as pale yellow liquid. As a result of gaschromatography analysis (column trade name: DB-1, column temperature: 100°C (0 minute)-(10 minutes) 280°C (15 minutes)), the purity was 99.5%.

### <Synthesis Example 6> (Synthesis of polymer compound A)

A polymer compound A was produced according to a method described in Japanese Patent Application National Publication (Laid-open) No. 2005-506439. That is, under a nitrogen atmosphere, a low molecular weight compound B (1.4731 g) and 2,7-dibromo-9,9-dioctylfluorene (1.6980 g) were dissolved in 30.0 mL of toluene previously bubbled with nitrogen. To the resultant solution was added palladium acetate (1.0 mg) and P(o-MeOPh)₃ (6.3 mg), then, 10.0 ml of a 20 wt% Et₄NOH aqueous solution was added, the mixture was heated up to 100°C, then, refluxed overnight. Into the resultant reaction liquid, methanol (465 ml) was poured to obtain 1.51 g of a polymer compound having a repeating unit represented by the following formula: 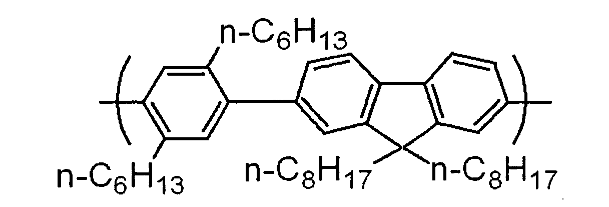 (hereinafter, referred to as "polymer compound A"). The polymer compound A had a polystyrene-equivalent number average molecular weight Mn of 5.1×10⁴ and a polystyrene-equivalent weight average molecular weight Mw of 101×10⁵.

### <Synthesis Example 7> (Synthesis of polymer compound B)

Under a nitrogen atmosphere, 2,7-bis(1,3,2-dioxaboloran-2-yl)-9,9-dioctylfluorene (5.20 g), bis(4-bromophenyl)-(4-s-butylphenyl)-amine(4.50 g), palladium acetate (2.2 mg), tri(2-methylphenyl)phosphine (15.1 mg), methyltrioctyl ammonium chloride (trade name: Aliquat (registered trademark) 336, 0.91g, manufactured by Aldrich) and toluene (70 ml) were mixed and heated at 105°C. Into the resultant reaction liquid, a 2M Na₂CO₃ aqueous solution (19 ml) was dropped, and the mixture was refluxed for 4 hours. After the reaction, phenylboric acid (121 mg) was added, and the mixture was refluxed further for 3 hours. Then, a sodium diethyldithiacarbamate aqueous solution was added, and the mixture was stirrred at 80°C for 4 hours. After cooling, the mixture was washed with water (60 ml) three times, with a 3 wt% acetic acid aqueous solution (60 ml) three times and with water (60 ml) three times, and passed through an alumina column and a silica gel column for purification. The resultant toluene solution was dropped into methanol (3 L), and the mixture was stirred for 3 hours, then, the resultant solid was removed and dried to obtain a polymer compound (alternate copolymer) having repeating units represented by the following formulae: 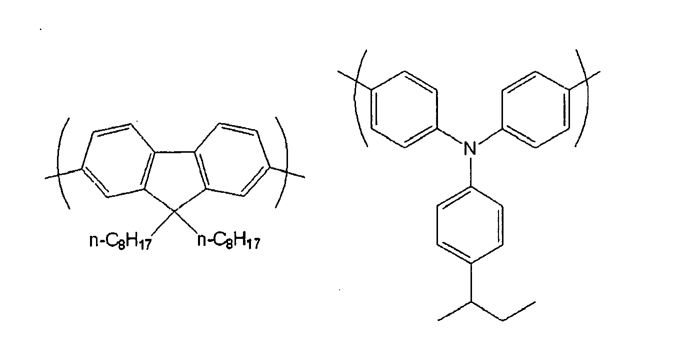 at a ratio of 50:50 (theoretical value from charged amount (molar ratio))(hereinafter, referred to as "polymer compound B"). The yielded amount of the polymer compound B was 5.25 g. The polymer compound B had a polystyrene-equivalent number average molecular weight of 1.2×10⁵ and a polystyrene-equivalent weight average molecular weight of 2.6×10⁵.

### <Synthesis Example 8> (Synthesis of polymer compound C)

A polymer compound C was produced according to a method described in Japanese Patent Application National Publication (Laid-open) No. 2005-506439. Under a nitrogen atmosphere, the low molecular weight compound B (0.9870 g), 2,7-dibromo-9,9-dioctylfluorene (0.9056 g) and the low molecular weight compound C(0.2205 g) were dissolved in 20.0 mL of toluene previously bubbled with nitrogen. Palladium acetate (0.7 mg) and P(o-MeOPh)₃ (4.2 mg) were added, and 6.6 ml of a 20 wt% Et₄NOH aqueous solution was added, and the mixture was heated up to 100°C, then, refluxed overnight. The resultant solution was poured into methanol (310 ml) to obtain 1.09 g of a polymer compound (random copolymer) having repeating units represented by the following formulae: 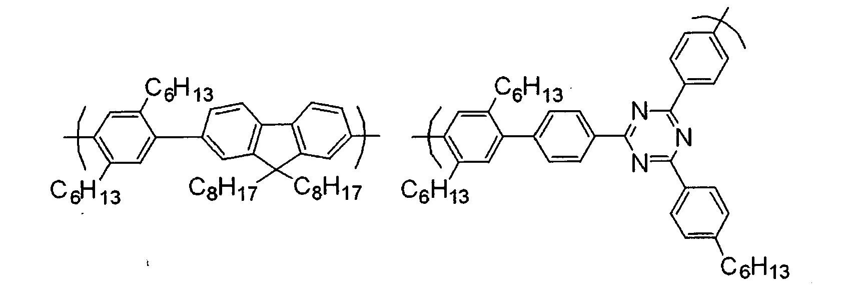 at a ratio of 80:20 (theoretical value from charged amount (molar ratio))(hereinafter, referred to as "polymer compound C"). The polymer compound C had a polystyrene-equivalent number average molecular weight of 1.2×10⁵ and a polystyrene-equivalent weight average molecular weight of 3.2×10⁵.

### <Synthesis Example 9> (Synthesis of light emitting material B)

Under an inert atmosphere, into a three-necked flask was added 9,10-dibromoanthracene (37.6 g, 0.11 mol), N-(4-t-butylphenyl)aniline (50.4 g, 0.22 mol), t-butoxysodium (25.8 g, 0.27 mol), [tris(dibenzylideneacetone)]dipalladium (2.1 g, 2.2 mmol), tri-t-butylphosphine (1.8 g, 9 mmol) and dehydrated toluene (91 mL), and the mixture was stirred at 100°C. Thereafter, the resultant solution was cooled down to room temperature, and a 1N hydrochloric acid aqueous solution (6.2 g) and methanol (1250 mL) were added while stirring, and the deposited crystal was filtrated, washed with MeOH and distilled water, and dried under reduced pressure to obtain a coarse product. This coarse product was recrystallized from hexane, to obtain 61 g of a light emitting material represented by the following formula: 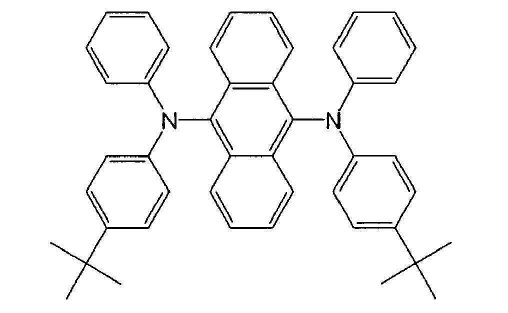 (yield 100%, HPLC area percentage 99.3%).
¹H-NMR (299.4 MHz, CDCl₃): 1.27 (s,18H), 6.86 (m,2H), 7.08 (m, 8H), 7.20 (m, 8H), 7.36 (m, 4H),8.21 (m, 4H)
LC-MS (APPI-MS (posi)): 625 [M+H]⁺

### <Example 1> (Fabrication of organic electroluminescent device 1)

On a glass substrate carrying thereon an ITO film having a thickness of 150 nm formed by a sputtering method, a suspension of poly(3,4)ethylenedioxythiophene/polystyrenesulfonic acid (manufactured by H. C. Starck, trade name:
BaytronP) (hereinafter, referred to as "Baytron P") was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the low molecular weight compound A, 2,2'-bipyridyl (manufactured by Aldrich) and the light emitting material A were dissolved at a concentration of 3.5 wt% in xylene (manufactured by KANTO Chemical Co., Inc.: for electronics industry (EL grade))(weight ratio of low molecular weight compound A/2,2'-bipyridyl/light emitting material A = 50/20/30). The resultant xylene solution was placed on a film of the polymer compound B, and a light emitting layer 1 was formed by a spin coating method to give a thickness of about 120 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer 1, then, aluminum was vapor-deposited with a thickness of about 100 nm on the barium layer, as a cathode. After vapor deposition, sealing thereof was performed using a glass substrate, to fabricate an organic electroluminescent device 1. The device constitution is as described below.

ITO/Baytron P (about 65 nm)/polymer compound B (about 10 nm)/light emitting layer 1 (about 120 nm)/Ba/Al

When a voltage of 8.4 V was applied on the organic electroluminescent device 1, light emission was observed with a light emission wavelength peak top at 520 nm and the luminance at this moment was 1103 cd/m². The luminance half life with an initial luminance of 3920 cd/m² was 256 hours. The obtained results are shown in Table 1.

### <Example 2> (Fabrication of organic electroluminescent device 2)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the polymer compound A, the low molecular weight compound A, 2,2'-bipyridyl (manufactured by Aldrich) and the light emitting material A were dissolved at a concentration of 2.5 wt% in xylene (manufactured by KANTO Chemical Co., Inc.: for electronics industry (EL grade))(weight ratio of polymer compound A/low molecular weight compound A/2,2'-bipyridyl/light emitting material A = 30/20/20/30). The resultant xylene solution (composition) was placed on a film of the polymer compound B, and a light emitting layer 2 was formed by a spin coating method to give a thickness of about 90 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer 2, then, aluminum was vapor-deposited with a thickness of about 100 nm on the barium layer, as a cathode. After vapor deposition, sealing thereof was performed using a glass substrate, to fabricate an organic electroluminescent device 2. The device constitution is as described below.

ITO/Baytron P (about 65 nm)/polymer compound B (about 10 nm)/light emitting layer 2 (about 90 nm)/Ba/Al

When a voltage of 6.0 V was applied on the organic electroluminescent device 2, light emission was observed with a light emission wavelength peak top at 520 nm and the luminance at this moment was 1140 cd/m². The luminance half life with an initial luminance of 4000 cd/m² was 105 hours. The obtained results are shown in Table 1.

### <Example 3> (Fabrication of organic electroluminescent device 3)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the polymer compound A, the low molecular weight compound A, 5,5'-dimethyl-2,2'-bipyridyl and the light emitting material A were dissolved at a concentration of 2.5 wt% in xylene (manufactured by KANTO Chemical Co., Inc.: for electronics industry (EL grade))(weight ratio of polymer compound A/low molecular weight compound A/5,5'-dimethyl-2,2'-bipyridyl/light emitting material A = 30/20/20/30). The resultant xylene solution (composition) was placed on a film of the polymer compound B, and a light emitting layer 3 was formed by a spin coating method to give a thickness of about 90 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer 3, then, aluminum was vapor-deposited with a thickness of about 100 nm on the barium layer, as a cathode. After vapor deposition, sealing thereof was performed using a glass substrate, to fabricate an organic electroluminescent device 3. The device constitution is as described below.

ITO/Baytron P (about 65 nm)/polymer compound B (about 10 nm)/light emitting layer 3 (about 90 nm)/Ba/Al

When a voltage of 6.0 V was applied on the organic electroluminescent device 3, light emission was observed with a light emission wavelength peak top at 520 nm and the luminance at this moment was 934 cd/m². The luminance half life with an initial luminance of 3970 cd/m² was 49 hours. The obtained results are shown in Table 1.

### <Example 4> (Fabrication of organic electroluminescent device 4)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the polymer compound C, 2,2'-bipyridyl (manufactured by Aldrich) and the light emitting material A were dissolved at a concentration of 2.0 wt% in xylene (manufactured by KANTO Chemical Co., Inc.: for electronics industry (EL grade))(weight ratio of polymer compound C/2,2'-bipyridyl/light emitting material A = 50/20/30).

The resultant xylene solution was placed on a film of the polymer compound B, and a light emitting layer 4 was formed by a spin coating method to give a thickness of about 100 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer 4, then, aluminum was vapor-deposited with a thickness of about 100 nm on the barium layer, as a cathode. After vapor deposition, sealing thereof was performed using a glass substrate, to fabricate an organic electroluminescent device 4. The device constitution is as described below.

ITO/Baytron P (about 65 nm)/polymer compound B (about 10 nm)/light emitting layer 4 (about 100 nm)/Ba/Al

When a voltage of 7.6 V was applied on the organic electroluminescent device 4, light emission was observed with a light emission wavelength peak top at 515 nm and the luminance at this moment was 961 cd/m². The luminance half life with an initial luminance of 4070 cd/m² was 155 hours. The obtained results are shown in Table 1.

### <Example 5> (Fabrication of organic electroluminescent device 5)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the polymer compound C, 5,5'-dimethyl-2,2'-bipyridyl and the light emitting material A were dissolved at a concentration of 2.0 wt% in xylene (manufactured by KANTO Chemical Co., Inc.: for electronics industry (EL grade))(weight ratio of polymer compound C/2,2'-bipyridyl/light emitting material A = 50/20/30). The resultant xylene solution was placed on a film of the polymer compound B, and a light emitting layer 5 was formed by a spin coating method to give a thickness of about 100 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer 5, then, aluminum was vapor-deposited with a thickness of about 100 nm on the barium layer, as a cathode. After vapor deposition, sealing thereof was performed using a glass substrate, to fabricate an organic electroluminescent device 5. The device constitution is as described below.

ITO/Baytron P (about 65 nm)/polymer compound B (about 10 nm)/light emitting layer 5 (about 100 nm)/Ba/Al

When a voltage of 7.4 V was applied on the organic electroluminescent device 5, light emission was observed with a light emission wavelength peak top at 520 nm and the luminance at this moment was 962 cd/m². The luminance half life with an initial luminance of 4050 cd/m² was 56 hours. The obtained results are shown in Table 1.

### <Example 6> (Fabrication of organic electroluminescent device 6)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the polymer compound C, the low molecular weight compound D and the light emitting material A were dissolved at a concentration of 2.0 wt% in xylene (manufactured by KANTO Chemical Co., Inc.: for electronics industry (EL grade))(weight ratio of polymer compound C/low molecular weight compound D/light emitting material A = 50/20/30). The resultant xylene solution was placed on a film of the polymer compound B, and a light emitting layer 6 was formed by a spin coating method to give a thickness of about 100 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer 6, then, aluminum was vapor-deposited with a thickness of about 100 nm on the barium layer, as a cathode. After vapor deposition, sealing thereof was performed using a glass substrate, to fabricate an organic electroluminescent device. The device constitution is as described below.

ITO/Baytron P (about 65 nm)/polymer compound B (about 10 nm)/light emitting layer 6 (about 100 nm)/Ba/Al

When a voltage of 7.4 V was applied on the organic electroluminescent device 6, light emission was observed with a light emission wavelength peak top at 520 nm and the luminance at this moment was 937 cd/m². The luminance half life with an initial luminance of 4000 cd/m² was 25 hours. The obtained results are shown in Table 1.

### <Example 7> (Fabrication of organic electroluminescent device 7)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the polymer compound A, the low molecular weight compound A, 2,2'-bipyridyl and the light emitting material B were dissolved at a concentration of 2.5 wt% in xylene (manufactured by KANTO Chemical Co., Inc.: for electronics industry (EL grade))(weight ratio of polymer compound A/low molecular weight compound A/2,2'-bipyridyl/light emitting material B = 55/20/20/5). The resultant xylene solution was placed on a film of the polymer compound B, and a light emitting layer 7 was formed by a spin coating method to give a thickness of about 100 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer 7, then, aluminum was vapor-deposited with a thickness of about 100 nm on the barium layer, as a cathode. After vapor deposition, sealing thereof was performed using a glass substrate, to fabricate an organic electroluminescent device 7. The device constitution is as described below.

ITO/Baytron P (about 65 nm)/polymer compound B (about 10 nm)/light emitting layer 7 (about 100 nm)/Ba/Al

When a voltage of 10.4 V was applied on the organic electroluminescent device 7, light emission was observed with a light emission wavelength peak top at 515 nm and the luminance at this moment was 1016 cd/m². The luminance half life with an initial luminance of 805 cd/m² was 107 hours. The obtained results are shown in Table 1.

### <Comparative Example 1> (Fabrication of organic electroluminescent device C1)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the low molecular weight compound A and the light emitting material A were dissolved at a concentration of 3.5 wt% in xylene (manufactured by KANTO Chemical Co., Inc.: for electronics industry (EL grade))(weight ratio of low molecular weight compound A/light emitting material A = 70/30). The resultant xylene solution (composition) was placed on a film of the polymer compound B, and a light emitting layer A was formed by a spin coating method to give a thickness of about 120 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer A, then, aluminum was vapor-deposited with a thickness of about 100 nm on the barium layer, as a cathode. After vapor deposition, sealing thereof was performed using a glass substrate, to fabricate an organic electroluminescent device C1. The device constitution is as described below.

ITO/Baytron P (about 65 nm)/polymer compound B (about 10 nm)/light emitting layer A (about 100 nm)/Ba/Al

When a voltage of 8.8 V was applied on the organic electroluminescent device C1, light emission was observed with a light emission wavelength peak top at 520 nm and the luminance at this moment was 980 cd/m². The luminance half life with an initial luminance of 3930 cd/m² was 29 hours. The obtained results are shown in Table 1.

### <Comparative Example 2> (Fabrication of organic electroluminescent device C2)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, 2,2'-bipyridyl and the light emitting material A were dissolved at a concentration of 5.0 wt% in xylene (manufactured by KANTO Chemical Co., Inc.: for electronics industry (EL grade))(weight ratio of 2,2'-bipyridyl/light emitting material A = 70/30). The resultant xylene solution (composition) was placed on a film of the polymer compound B, and a light emitting layer B was formed by a spin coating method to give a thickness of about 50 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer B, then, aluminum was vapor-deposited with a thickness of about 100 nm on the barium layer, as a cathode. After vapor deposition, sealing thereof was performed using a glass substrate, to fabricate an organic electroluminescent device C2. The device constitution is as described below.

ITO/Baytron P (about 65 nm)/polymer compound B (about 10 nm)/light emitting layer B (about 100 nm)/Ba/Al

When a voltage of 11.6 V was applied on the organic electroluminescent device C2, light emission was observed with a light emission wavelength peak top at 520 nm and the luminance at this moment was 431 cd/m². The luminance half life with an initial luminance of 67 cd/m² was 6 hours.

The obtained results are shown in Table 1.

### <Comparative Example 3> (Fabrication of organic electroluminescent device C3)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the polymer compound A, the low molecular weight compound A and the light emitting material A were dissolved at a concentration of 2.5 wt% in xylene (manufactured by KANTO Chemical Co., Inc.: for electronics industry (EL grade))(weight ratio of polymer compound A/low molecular weight compound A/light emitting material A = 50/20/30). The resultant xylene solution (composition) was placed on a film of the polymer compound B, and a light emitting layer C was formed by a spin coating method to give a thickness of about 90 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer C, then, aluminum was vapor-deposited with a thickness of about 100 nm on the barium layer, as a cathode. After vapor deposition, sealing thereof was performed using a glass substrate, to fabricate an organic electroluminescent device C3. The device constitution is as described below.

ITO/Baytron P (about 65 nm)/polymer compound B (about 10 nm)/light emitting layer C (about 90 nm)/Ba/Al

When a voltage of 6.8 V was applied on the organic electroluminescent device C3, light emission was observed with a light emission wavelength peak top at 515 nm and the luminance at this moment was 1039 cd/m². The luminance half life with an initial luminance of 4010 cd/m² was 3 hours. The obtained results are shown in Table 1.

### <Comparative Example 4> (Fabrication of organic electroluminescent device C4)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the polymer compound C and the light emitting material A were dissolved at a concentration of 1.3 wt% in xylene (manufactured by KANTO Chemical Co., Inc.: for electronics industry (EL grade))(weight ratio of polymer compound C/light emitting material A = 70/30). The resultant xylene solution (composition) was placed on a film of the polymer compound B, and a light emitting layer D was formed by a spin coating method to give a thickness of about 100 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer D, then, aluminum was vapor-deposited with a thickness of about 100 nm on the barium layer, as a cathode. After vapor deposition, sealing thereof was performed using a glass substrate, to fabricate an organic electroluminescent device C4. The device constitution is as described below.

ITO/Baytron P (about 65 nm)/polymer compound B (about 10 nm)/light emitting layer D (about 100 nm)/Ba/Al

When a voltage of 7.2 V was applied on the organic electroluminescent device C4, light emission was observed with a light emission wavelength peak top at 515 nm and the luminance at this moment was 949 cd/m². The luminance half life with an initial luminance of 4020 cd/m² was 5 hours. The obtained results are shown in Table 1.

### <Comparative Example 5> (Fabrication of organic electroluminescent device C5)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the polymer compound A, 2,2'-bipyridyl and the light emitting material A were dissolved at a concentration of 2.2 wt% in xylene (manufactured by KANTO Chemical Co., Inc.: for electronics industry (EL grade))(weight ratio of polymer compound A/2,2'-bipyridyl/light emitting material A = 50/20/30). The resultant xylene solution (composition) was placed on a film of the polymer compound B, and a light emitting layer E was formed by a spin coating method to give a thickness of about 100 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer E, then, aluminum was vapor-deposited with a thickness of about 100 nm on the barium layer, as a cathode. After vapor deposition, sealing thereof was performed using a glass substrate, to fabricate an organic electroluminescent device C4. The device constitution is as described below.

ITO/Baytron P (about 65 nm)/polymer compound B (about 10 nm)/light emitting layer E (about 100 nm)/Ba/Al

When a voltage of 12.0 V was applied on the organic electroluminescent device C5, light emission was observed with a light emission wavelength peak top at 520 nm and the luminance at this moment was 896 cd/m². The luminance half life with an initial luminance of 4030 cd/m² was 4 hours. The obtained results are shown in Table 1.

### <Comparative Example 6> (Fabrication of organic electroluminescent device C6)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the polymer compound A, the low molecular weight compound A and the light emitting material B were dissolved at a concentration of 2.0 wt% in xylene (manufactured by KANTO Chemical Co., Inc.: for electronics industry (EL grade))(weight ratio of polymer compound A/low molecular weight compound A/light emitting material B = 75/20/5).

The resultant xylene solution was placed on a film of the polymer compound B, and a light emitting layer F was formed by a spin coating method to give a thickness of about 100 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer F, then, aluminum was vapor-deposited with a thickness of about 100 nm on the barium layer, as a cathode. After vapor deposition, sealing thereof was performed using a glass substrate, to fabricate an organic electroluminescent device. The device constitution is as described below.

ITO/Baytron P (about 65 nm)/polymer compound B (about 10 nm)/light emitting layer F (about 100 nm)/Ba/Al

When a voltage of 10.8 V was applied on the organic electroluminescent device C6, light emission was observed with a light emission wavelength peak top at 515 nm and the luminance at this moment was 981 cd/m². The luminance half life with an initial luminance of 802 cd/m² was 13 hours. The obtained results are shown in Table 1.

### <Comparative Example 7> (Fabrication of organic electroluminescent device C7)

On a glass substrate carrying thereon an ITO film having a thickness of 150 nm formed by a sputtering method, a suspension of Baytron P was placed, and film-formed by a spin coating method to give a thickness of about 65 nm, and the film was dried on a hot plate at 200°C for 10 minutes. Then, the polymer compound B was dissolved at a concentration of 0.5 wt% in xylene (manufactured by KANTO Chemical Co., Inc.: for electronics industry (EL grade)), and the resultant xylene solution was placed on a film of Baytron P, film-formed by a spin coating method, then, dried at 180°C for 15 minutes under a nitrogen atmosphere in which the oxygen concentration and the water concentration were not more than 10 ppm (weight base). Next, the polymer compound A, 2,2'-bipyridyl and the light emitting material B were dissolved at a concentration of 2.0 wt% in xylene (manufactured by KANTO Chemical Co., Inc.: for electronics industry (EL grade))(weight ratio of polymer compound A/2,2'-bipyridyl/light emitting material B = 75/20/5). The resultant xylene solution was placed on a film of the polymer compound B, and a light emitting layer G was formed by a spin coating method to give a thickness of about 100 nm. The film was dried at 90°C for 10 minutes under a nitrogen atmosphere in which the oxygen concentration and the water concentration were not more than 10 ppm (weight base). The pressure was reduced to 1.0×10⁻⁴ Pa or lower, then, barium was vapor-deposited with a thickness of about 5 nm on a film of the light emitting layer G, then, aluminum was vapor-deposited with a thickness of about 100 nm on the barium layer, as a cathode. After vapor deposition, sealing thereof was performed using a glass substrate, to fabricate an organic electroluminescent device. The device constitution is as described below.

ITO/Baytron P (about 65 nm)/polymer compound B (about 10 nm)/light emitting layer G (about 100 nm)/Ba/Al

Even if a voltage of 12.0 V was applied on the organic electroluminescent device C7, the luminance at this moment was 10 cd/m² or less. The luminance half life with an initial luminance of 312 cd/m² was less than 1 hour. The obtained results are shown in Table 1.

**Table 1**

| | | formulation of composition used for fabriration of light emitting layer (weight ratio) | luminance half life (hr) | initial luminance (cd/m²) |
|---|---|---|---|---|
| low molecular weight compound single use system | Example 1 | low molecular weight compound A/2,2'-bipyridyl/light emitting material A = 50/20/30 | 256 | 3920 |
| | Comparative Example 1 | low molecular weight compound A/light emitting material A = 70/30 | 29 | 3930 |
| | Comparative Example 2 | 2,2'-bipyridyl/light emitting material A = 70/30 | 6 | 67 |
| polymer mixed use system | Example 2 | polymer compound A/low molecular weight compound A/2,2'-bipyridyl/light emitting material A = 30/20/20/30 | 105 | 4000 |
| | Example 3 | polymer compound A/low molecular weight compound A/5,5'-dimethyl-2,2'-bipyridyl/light emitting material A = 30/20/20/30 | 49 | 3970 |
| | Example 4 | polymer compound C/2,2'-bipyridyl/light emitting material A = 50/20/30 | 155 | 4070 |
| | Example 5 | polymer compound C/5,5'-dimethyl-2,2'-bipyridyl/light emitting material A = 50/20/30 | 56 | 4050 |
| | Example 6 | polymer compound C/low molecular weight compound D/light emitting material A = 50/20/30 | 25 | 4000 |
| | Example 7 | polymer compound A/low molecular weight compound A/2,2'-bipyridyl/light emitting material B = 55/20/20/5 | 107 | 805 |
| | Comparative Example 3 | polymer compound A/low molecular weight compound A/light emitting material A = 50/20/30 | 3 | 4010 |
| | Comparative Example 4 | polymer compound C/light emitting material A = 70/30 | 5 | 4020 |
| | Comparative Example 5 | polymer compound A/2,2'-bipyridyl/light emitting material A = 50/20/30 | 4 | 4030 |
| | Comparative Example 6 | polymer compound A/low molecular weight compound A/light emitting material B = 75/20/5 | 13 | 802 |
| | Comparative Example 7 | polymer compound A/2,2'-bipyridyl/light emitting material B = 75/20/5 | less than 1 | 312 |

### <Example 8> (Fabrication of organic electroluminescent device 8) .

An organic electroluminescent device was fabricated in the same manner as in Example 2 excepting that the drying temperature of the film of the light emitting layer 2 was changed from 90°C to 50°C in Example 2 (hereinafter, referred to as "organic electroluminescent device 8").

The time in measuring the luminance half life under a constant current at an initial luminance of 4000 cd/m² for the organic electroluminescent device 8 was 40% with respect to the luminance half life in Example 2. The obtained results are shown in Table 2.

### <Example 9> (Fabrication of organic electroluminescent device 9)

An organic electroluminescent device was fabricated in the same manner as in Example 2 excepting that the drying temperature of the film of the light emitting layer 2 was changed from 90°C to 130°C in Example 2 (hereinafter, referred to as "organic electroluminescent device 9").

The time in measuring the luminance half life under a constant current at an initial luminance of 4000 cd/m² for the organic electroluminescent device 9 was 42% with respect to the luminance half life in Example 2. The obtained results are shown in Table 2.

**Table 2**

| | heating temperature of light emitting layer (°C) | luminance half life (normalized) |
|---|---|---|
| Example 2 | 90 | 100 |
| Example 8 | 50 | 40 |
| Example 9 | 130 | 42 |

### Industrial Applicability

The composition (organic material) of the present invention is capable of giving an organic electroluminesence device having a longer luminance half life, when used for production of the organic electroluminesence device. The composition of the present invention is useful also as a hole transporting material or electron transporting material.

## Claims

1. A composition comprising:
a compound represented by the following formula (1): 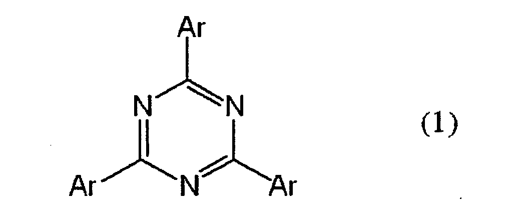 wherein each Ar represents an aryl group optionally having a substituent or a mono-valent heterocyclic group optionally having a substituent,where three Ars may be the same or different,
or a compound having a residue of a compound represented by the above-described formula (1), and
a compound represented by the following formula (2): 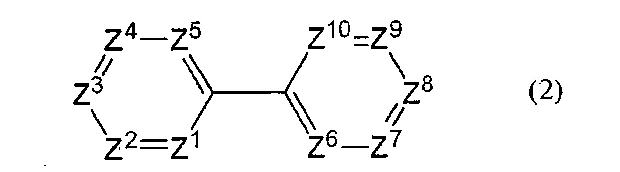 wherein one member of Z¹, Z² and Z³ represents -N= and two remainders thereof each represent -C(R')=; Z⁴ and Z⁵ each represent -C(R')=; one member of Z⁶, Z⁷ and Z⁸ represents -N= and two remainders thereof each represent -C(R')=; Z⁹ and Z¹⁰ each represent -C(R')=; R' represents a hydrogen atom, an alkyl group optionally having a substituent, an alkoxy group optionally having a substituent, an alkylthio group optionally having a substituent, an aryl group optionally having a substituent, an aryloxy group optionally having a substituent, an arylthio group optionally having a substituent, an alkenyl group optionally having a substituent, an alkynyl group optionally having a substituent, an amino group optionally having a substituent, a silyl group optionally having a substituent, a halogen atom, an acyl group optionally having a substituent, an acyloxy group optionally having a substituent, a mono-valent heterocyclic group optionally having a substituent, a heterocyclic thio group optionally having a substituent, an imine residue, an amide group optionally having a substituent, an acid imide group, a carboxyl group, a nitro group, or a cyano group;
eight -C(R')= groups may be the same or different;
when Z² and Z³ each represent -C(R')=, two R's contained in Z² and Z³ may be combined together to form a benzene ring, and when Z³ represents -C(R')=, two R's contained in Z³ and z⁴ may be combined together to form a benzene ring and two R's contained in Z⁴ and Z⁵ may be combined together to form a benzene ring, providing that two or more combinations of z² and Z³, Z³ and Z⁴, and Z⁴ and Z⁵ do not simultaneously form a benzene ring; when Z⁷ and Z⁸ each represent -C(R')=, two R's contained in Z⁷ and Z⁸ may be combined together to form a benzene ring, and when Z⁸ represents -C(R')=, two R's contained in Z⁸ and Z⁹ may be combined together to form a benzene ring and two R's contained in Z⁹ and Z¹⁰ may be combined together to form a benzene ring, providing that two or more combinations of Z⁷ and Z⁸, Z⁸ and Z⁹, and Z⁹ and Z¹⁰ do not simultaneously form a benzene ring; the benzene ring formed by mutual combination of two R's optionally has a substituent,
or a compound having a residue of a compound represented by the above-described formula (2).

2. The composition according to Claim 1, wherein R' in the above-described formula (2) represents a hydrogen atom, an alkyl group optionally substituted by a fluorine atom, an alkoxy group optionally substituted by a fluorine atom, an aryl group optionally substituted by a fluorine atom, an aryloxy group optionally substituted by a fluorine atom, an arylalkyl group optionally substituted by a fluorine atom, an arylalkoxy group optionally substituted by a fluorine atom, an arylalkenyl group optionally substituted by a fluorine atom, an arylalkynyl group optionally substituted by a fluorine atom, an amino group optionally substituted by a fluorine atom, a substituted amino group optionally substituted by a fluorine atom, a halogen atom, an acyl group optionally substituted by a fluorine atom, an acyloxy group optionally substituted by a fluorine atom, a mono-valent heterocyclic group optionally substituted by a fluorine atom, a carboxyl group, a nitro group, or a cyano group.

3. The composition according to Claim 1 or 2 comprising a compound represented by the above-described formula (1) and a compound represented by the above-described formula (2).

4. The composition according to Claim 3, wherein said Ar represents a phenyl group optionally having a substituent or a biphenyl group optionally having a substituent.

5. The composition according to Claim 4, wherein said Ar represents a phenyl group substituted by an alkyl group having 1 to 12 carbon atoms or a biphenyl group substituted by an alkyl group having 1 to 12 carbon atoms.

6. The composition according to Claim 1 or 2 comprising a compound having a residue of a compound represented by the above-described formula (1) and a compound represented by the above-described formula (2) or a compound having a residue of a compound represented by the above-described formula (2), wherein the compound having a residue of a compound represented by the above-described formula (1) is a polymer compound having a repeating unit containing a residue of a compound represented by the above-described formula (1).

7. The composition according to Claim 1 or 2 comprising a compound represented by the above-described formula (1) or a compound having a residue of a compound represented by the above-described formula (1) and a compound having a residue of a compound represented by the above-described formula (2), wherein the compound having a residue of a compound represented by the above-described formula (2) is a polymer compound having a repeating unit containing a residue of a compound represented by the above-described formula (2).

8. The composition according to Claim 1 or 2 comprising a compound having a residue of a compound represented by the above-described formula (1) and a compound having a residue of a compound represented by the above-described formula (2), wherein the compound having a residue of a compound represented by the above-described formula (1) is a polymer compound having a repeating unit containing a residue of a compound represented by the above-described formula (1), and the compound having a residue of a compound represented by the above-described formula (2) is a polymer compound having a repeating unit containing a residue of a compound represented by the above-described formula (2).

9. The composition according to Claim 6 or 8, wherein in the polymer compound having a repeating unit containing a residue of a compound represented by the above-described formula (1), the repeating unit is represented by the following formula (3): 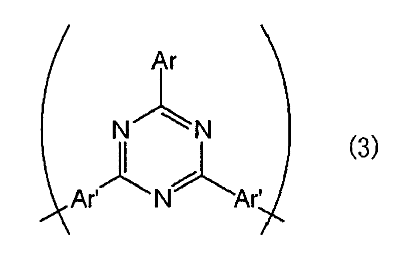 wherein Ar has the same meaning as described above; each Ar' represents an arylene group optionally having a substituent or a di-valent heterocyclic group optionally having a substituent,where two Ar's may be the same or different.

10. The composition according to Claim 9, wherein said Ar represents a phenyl group optionally having a substituent and each Ar' represents a 1,4-phenylene group optionally having a substituent.

11. The composition according to any one of Claims 6 to 9, wherein said R' represents a hydrogen atom or alkyl group.

12. The composition according to any one of Claims 1 to 10, wherein the compound represented by the above-described formula (2) is 2,2'-bipyridyl, 5-dimethyl-2,2'-bipyridyl, 5,5'-dimethyl-2,2'-bipyridyl, 3,3'-bipyridyl or 4,4'-bipyridyl.

13. The composition according to Claim 7 or 8, wherein in the polymer compound having a repeating unit containing a residue of a compound represented by the above-described formula (2), the repeating unit is a repeating unit represented by the following formula (4): 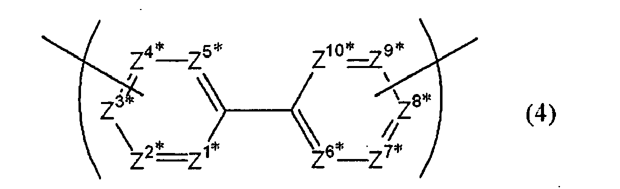 wherein one member of Z¹*, Z²* and Z³* represents -N= and two remainders thereof each represent -C(R")=; Z⁴* and Z⁵* each represent -C(R")=; one member of Z⁶*, Z⁷* and Z⁸* represents -N= and two remainders thereof each represent -C(R")=; Z⁹* and Z¹⁰* each represent -C(R'')=; R" represents a hydrogen atom, an alkyl group, an alkoxy group, an aryl group, an aryloxy group, an arylalkyl group, an arylalkoxy group, an arylalkenyl group, an arylalkynyl group, an amino group, a substituted amino group, a halogen atom, an acyl group, an acyloxy group, a mono-valent heterocyclic group, a carboxyl group, a substituted carboxyl group, a nitro group, or a cyano group, and one R" contained in Z¹*, Z²*, Z³*, Z⁴* and Z⁵* represents a connecting bond, and one R" contained in Z⁶*, Z⁷*, Z⁸*, Z⁹* and Z¹⁰* represents a connecting bond; one or some or all of the hydrogen atoms contained in the group represented by R" may be substituted by a fluorine atom; eight -C(R'')= groups may be the same or different; when Z²* and Z³* each represent -C(R")=, two R"s contained in Z²* and Z³* may be combined together to form a benzene ring, and when Z³* represents -C(R")=, two R"s contained in Z³* and Z⁴* may be combined together to form a benzene ring and two R"s contained in Z⁴* and Z⁵* may be combined together to form a benzene ring, providing that two or more combinations of Z²* and Z³*, Z³* and Z⁴*, and Z⁴* and Z⁵* do not simultaneously form a benzene ring; when Z⁷* and Z⁸* each represent -C(R")=, two R"s contained in Z⁷* and Z⁸* may be combined together to form a benzene ring, and when Z⁸* represents -C(R")=, two R"s contained in Z⁸* and Z⁹* may be combined together to form a benzene ring and two R''s contained in Z⁹* and Z¹⁰* may be combined together to form a benzene ring, providing that two or more combinations of Z⁷* and Z⁸*, Z⁸* and Z⁹*, and Z⁹* and Z¹⁰* do not simultaneously form a benzene ring; the benzene ring formed by mutual combination of two R"s optionally has a substituent or a repeating unit represented by the following formula (5) : 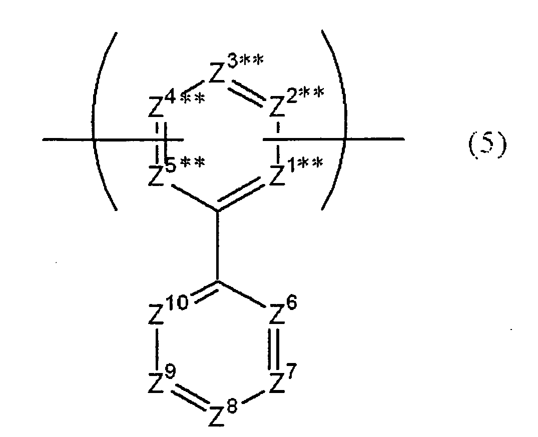 wherein one member of Z¹**, Z²** and Z³** represents -N= and two remainders thereof each represent -C(R'")=; Z⁴** and Z⁵** each represent -C(R'")=; R'" represents a hydrogen atom, an alkyl group, an alkoxy group, an aryl group, an aryloxy group, an arylalkyl group, an arylalkoxy group, an arylalkenyl group, an arylalkynyl group, an amino group, a substituted amino group, a halogen atom, an acyl group, an acyloxy group, a mono-valent heterocyclic group, a carboxyl group, a substituted carboxyl group, a nitro group, or a cyano group, and two R'"s contained in Z¹**, z²**, Z³**, Z⁴** and Z⁵** each represent a connecting bond;
Z⁶, Z⁷, Z⁸, Z⁹ and Z¹⁰ each have the same meaning as described above; one or some or all of the hydrogen atoms contained in the group represented by R' and R'" may be substituted by a fluorine atom; four -C(R')= groups may be the same or different; four -C(R'")= groups may be the same or different; when Z²** and Z³** each represent - C(R'")=, two R'"s contained in Z²** and Z³** may be combined together to form a benzene ring, and when Z³** represents -C(R"')=, two R"'s contained in Z³** and Z⁴** may be combined together to form a benzene ring and two R"'s contained in Z⁴** and Z⁵** may be combined together to form a benzene ring, providing that two or more combinations of Z²** and Z³**, Z³** and Z⁴**, and Z⁴** and Z⁵** do not simultaneously form a benzene ring; when Z⁷ and Z⁸ each represent -C(R')=, two R's contained in Z⁷ and Z⁸ may be combined together to form a benzene ring, and when Z⁸ represents -C(R')=, two R's contained in Z⁸ and Z⁹ may be combined together to form a benzene ring and two R's contained in Z⁹ and Z¹⁰ may be combined together to form a benzene ring, providing that two or more combinations of Z⁷ and Z⁸, Z⁸ and Z⁹, and Z⁹ and Z¹⁰ do not simultaneously form a benzene ring;the benzene ring formed by mutual combination of two R's optionally has a substituent, and the benzene ring formed by mutual combination of two R"'s optionally has a substituent.

14. The composition according to Claim 13, wherein R" which is not a connecting bond is a hydrogen atom or an alkyl group.

15. The composition according to Claim 13, wherein R'" which is not a connecting bond is a hydrogen atom or an alkyl group.

16. The composition according to any one of Claims 1 to 15 further comprising at least one material selected from the group consisting of light emitting materials, hole transporting materials and electron transporting materials.

17. The composition according to any one of Claims 1 to 16 further comprising an organic solvent.

18. An organic electroluminescent device obtained by using the composition according to any one of Claims 1 to 17.

19. A planar light source equipped with the organic electroluminescent device according to Claim 18.

20. A display comprising the organic electroluminescent device according to Claim 18.

21. A film obtained by using the composition according to any one of Claims 1 to 17.

22. A method of producing an organic electroluminescent device, the method comprising heating the film according to Claim 21 at 50 to 200°C.
